# FASCICULE DE BREVET EUROPEEN

(11) **EP 3 049 540 B1**
(45) Date de publication et mention de la délivrance du brevet: **18.04.2018**
(21) Numéro de dépôt: 14790659.8
(22) Date de dépôt: 24.09.2014
(51) Int. Cl.: C12R 1/465, A01N 63/00, A01N 63/02, C05F 11/08, C12N 1/20

(54) **UTILISATIONS EN AGRICULTURE D'UNE NOUVELLE BACTÉRIE DU GENRE STREPTOMYCES**
LANDWIRTSCHAFTLICHE VERWENDUNG EINES NEUARTIGEN BAKTERIUMS DER GATTUNG STREPTOMYCES
AGRICULTURAL USES OF A NOVEL BACTERIUM OF THE GENUS STREPTOMYCES

(30) Priorité: 24.09.2013 FR 1359181
(43) Date de publication de la demande: 03.08.2016
(73) Titulaire: Agronutrition, 31390 Carbonne (FR); Centre National de la Recherche Scientifique (C.N.R.S.), 75794 Paris Cedex 16 (FR); Université Paul Sabatier Toulouse III, 31400 Toulouse (FR)
(72) Inventeur: ERRAKHI, Rafik, Benguerir (MA); ATTIA, Faouzi, F - 31450 Ayguesvives (FR); CABANES, Cédric, F-31320 Pechabou (FR); DUMAS, Bernard, F-31850 Montrabe (FR); VERGNES, Sophie, F-31790 Saint Sauveur (FR)
(74) Mandataire: Cabinet BARRE LAFORGUE & associés
(86) Numéro de dépôt international: PCT/FR2014/052388
(87) Numéro de publication internationale: WO 2015/044585

(56) Documents cités:
- WO-A2-2009/156687
- WO-A2-2009/156688
- WO-A2-2012/016140
- DE-A1- 10 324 193
- KR-A- 20100 055 364
- DATABASE EMBL [Online] 31 janvier 2013 (2013-01-31), "Streptomyces sp. AgN23 16S ribosomal RNA gene, partial sequence.", XP002722625, extrait de EBI accession no. EMBL:JN104730 Database accession no. JN104730
- HYE YOON KIM ET AL: "Identification of antifungal niphimycin from Streptomyces sp. KP6107 by screening based on adenylate kinase assay", JOURNAL OF BASIC MICROBIOLOGY, vol. 53, no. 7, 23 juillet 2013 (2013-07-23), pages 581-589, XP055111207, ISSN: 0233-111X, DOI: 10.1002/jobm.201200045 -& DATABASE EMBL [Online] 15 mai 2011 (2011-05-15), "Streptomyces hygroscopicus subsp. enhygrus strain KP6107 16S ribosomal RNA gene, partial sequence.", XP002722624, extrait de EBI accession no. EMBL:HQ441251 Database accession no. HQ441251
- SOUAD LOQMAN ET AL: "Antagonistic actinomycetes from Moroccan soil to control the grapevine gray mold", WORLD JOURNAL OF MICROBIOLOGY AND BIOTECHNOLOGY, KLUWER ACADEMIC PUBLISHERS, DO, vol. 25, no. 1, 4 octobre 2008 (2008-10-04), pages 81-91, XP019650434, ISSN: 1573-0972
- S SRIVIDYA ET AL: "Streptomyces sp. 9p as effective biocontrol against chilli soilborne fungal phytopathogens", EUROPEAN JOURNAL OF EXPERIMENTAL BIOLOGY, vol. 2, no. 1, 2012, pages 163-173, XP055111252,
- S. LOQMAN ET AL: "Streptomyces thinghirensis sp. nov., isolated from rhizosphere soil of Vitis vinifera", INTERNATIONAL JOURNAL OF SYSTEMATIC AND EVOLUTIONARY MICROBIOLOGY, vol. 59, no. 12, 30 juillet 2009 (2009-07-30), pages 3063-3067, XP055164561, ISSN: 1466-5026, DOI: 10.1099/ijs.0.008946-0
- Q. TU ET AL: "Strain/Species-Specific Probe Design for Microbial Identification Microarrays", APPLIED AND ENVIRONMENTAL MICROBIOLOGY, vol. 79, no. 16, 7 juin 2013 (2013-06-07), pages 5085-5088, XP055111359, ISSN: 0099-2240, DOI: 10.1128/AEM.01124-13
- MIKA T. TARKKA ET AL: "Plant behavior upon contact with streptomycetes", PLANT SIGNALING & BEHAVIOR, vol. 3, no. 11, novembre 2008 (2008-11), pages 917-919, XP0055164498, DOI: 10.4161/psb.5996

### SEQUENCE LISTING

<110> AGRONUTRITION CENTRE NATIONAL DE LA RECHERCHE SCIENTIFIQUE UNIVERSITE PAUL SABATIER TOULOUSE III
<120> UTILISATIONS D'UNE NOUVELLE SOUCHE DE STREPTOMYCES
<150> FR1359181
   <151> 2013-09-24
<160> 12
<170> BiSSAP 1.0
<210> 1
   <211> 1336
   <212> DNA
   <213> Streptomyces
<220>
   <221> source
   <222> 1..1336
   <223> /mol_type="DNA" /organism="Streptomyces"
<400> 1
   tagtggcgaa cgggtgagta acacgtgggc aatctgccct gcactctggg acaagccctg      60
   gaaacggggt ctaataccgg atatgacacg ctcccgcatg ggatgcgtgt ggaaagctcc     120
   ggcggtgcag gatgagcccg cggcctatca gcttgttggt ggggtgatgg cctaccaagg     180
   cgacgacggg tagccggcct gagagggcga ccggccacac tgggactgag acacggccca     240
   gactcctacg ggaggcagca gtggggaata ttgcacaatg ggcgaaagcc tgatgcagcg     300
   acgccgcgtg agggatgacg gccttcgggt tgtaaacctc tttcagcagg gaagaagcga     360
   gagtgacggt acctgcagaa gaagcgccgg ctaactacgt gccagcagcc gcggtaatac     420
   gtagggcgca agcgttgtcc ggaattattg ggcgtaaaga gctcgtaggc ggcttgtcgc     480
   gtcggatgtg aaagcccggg gcttaacccc gggtctgcat tcgatacggg caggctagag     540
   ttcggtaggg gagatcggaa ttcctggtgt agcggtgaaa tgcgcagata tcaggaggaa     600
   caccggtggc gaaggcggat ctctgggccg atactgacgc tgaggagcga aagcgtgggg     660
   agcgaacagg attagatacc ctggtagtcc acgccgtaaa cgttgggaac taggtgtggg     720
   cgacattcca cgtcgtccgc gccgcagcta acgcattaag ttccccgcct ggggagtacg     780
   gccgcaaggc taaaactcaa aggaattgac ggggggcccg cacaagcggc ggagcatgtg      840
   gcttaattcg acgcaacgcg aagaacctta ccaaggcttg acatacaccc ggaaacctct     900
   ggagacaggg gccccccttg tggtcggtgt acaggtggtg catggcttgt cgtcagctcg      960
   tgtcgtgaga tgttgggtta agtccccgca acgagcgcaa cccttgttct gtgttgccag    1020
   catgcctttc gggggntgat ggggacttnc acaggagact gccggggtca actcggagga    1080
   aggtggggac gacgtcaagt catcatgccc cttatgtctt gggctgcaca cgtgctacaa    1140
   tggccggtac aatgagctgc gaagccgtga ggtggagcga atctcaaaaa gccggtctca    1200
   gttcggattg gggtctgcaa ctcgacccca tgaagtcgga gtcgctagta atcgcagatc     1260
   agcattgctg cggtgaatac gttcccgggc cttgtacaca ccgcccgtca cgtcacgaaa    1320
   gtcggtaaca cctgaa                                                    1336
<210> 2
   <211> 20
   <212> DNA
   <213> Streptomyces
<220>
   <221> source
   <222> 1..20
   <223> /mol_type="DNA" /note="amorce universelle 27 f" /organism="Streptomyces"
<400> 2
   agagtttgat cctggctcag 20
<210> 3
   <211> 19
   <212> DNA
   <213> Streptomyces
<220>
   <221> source
   <222> 1..19
   <223> /mol_type="DNA" /note="amorce universelle 1492r" /organism="Streptomyces"
<400> 3
   ggttaccttg ttacgactt 19
<210> 4
   <211> 24
   <212> DNA
   <213> Streptomyces
<220>
   <221> source
   <222> 1..24
   <223> /mol_type="DNA" /note="amorce spécifique PR-1" /organism="Streptomyces"
<400> 4
   ctggctattc tcgattttta atcg 24
<210> 5
   <211> 24
   <212> DNA
   <213> Streptomyces
<220>
   <221> source
   <222> 1..24
   <223> /mol_type="DNA" /note="amorce spécifique PR-1 r" /organism="Streptomyces"
<400> 5
   tcctgcatat gatgctcctt attg 24
<210> 6
   <211> 24
   <212> DNA
   <213> Streptomyces
<220>
   <221> source
   <222> 1..24
   <223> /mol_type="DNA" /note="amorce EF1 f" /organism="Streptomyces"
<400> 6
   atgccccagg acatcgtgat ttca 24
<210> 7
   <211> 24
   <212> DNA
   <213> Streptomyces
<220>
   <221> source
   <222> 1..24
   <223> /mol_type="DNA" /note="amorce EF1 r" /organism="Streptomyces"
<400> 7
   ttggcggcac ccttagctgg atca 24
<210> 8
   <211> 3483
   <212> DNA
   <213> Streptomyces
<220>
   <221> source
   <222> 1..3483
   <223> /mol_type="DNA" /note="rpoB" /organism="Streptomyces"
<400> 8
   ttggccgcct cgcgcaacgc ctcgactgcc aatacgaaca atggcgccag caccgccccg 60
   ctgcgcatct cctttgcgaa gatcagggag cctctcgagg ttccgaacct cctcgcgctg 120
   cagaccgaga gcttcgattg gctgctcggc aatgccgcct ggaaggctcg cgtcgaggct 180
   gcgctggaca gcggtcagga cgtccccacc aagtccggtc tggaagagat cttcgaggag 240
   atctccccga tcgaggactt ctccgggtcg atgtccctga ctttccgtga tcaccgtttc 300
   gagccgccga agaactcgat cgacgagtgc aaggagcgtg acttcaccta cgccgctccg 360
   ctcttcgtca cggccgagtt caccaacaac gagaccggcg agatcaagtc ccagacggtc 420
   ttcatgggcg acttcccgct catgaccgac aagggcacct tctgcatcaa cggcaccgag 480
   cgtgtcgtcg tctcgcagct ggtccgctcg ccgggtgtct acttcgactc ctccatcgac 540
   aagacgtccg acaaggacat cttctccgtc aaggtcatcc cgtcccgggg tgcctggctg 600
   gagatggaga tcgacaagcg tgacatggtc ggtgtgcgta tcgaccgcaa gcgcaagcag 660
   tccgtcaccg ttctcctgaa ggctctcggc tggacgaccg agcagatcct ggaggagttc 720
   ggcgagtacg agtcgatgcg cgccaccctg gagaaggacc acacccaggg ccaggacgac 780
   gcgctgctcg acatctaccg caagctgcgt ccgggcgagc cccccacacg ggaggccgcg 840
   cagacgctgc tcgagaacct ctacttcaac ccgaagcgct acgacctcgc gaaggtcggc 900
   cgctacaagg tcaacaagaa gctgggttcg gccgctccgc tggacgcggg cgtcctgacg 960
   gtcgaggacg tcatcgcctc gatcaagtac ctggtgaagc tgcacgccgg tgagaccgag 1020
   accgtcgggg acaacggcca gtccgtggtc gtcgagaccg acgacatcga ccacttcggc 1080
   aaccgccgta tccgtaacgt cggcgagctg atccagaacc aggtccgcac gggtctggcc 1140
   cgtatggagc gcgtcgtgcg tgagcgcatg acgactcagg acgtcgaggc gatcacgccg 1200
   cagaccctga tcaacatccg gccggtcgtc gcctccatca aggagttctt cggcaccagc 1260
   cagctgtcgc agttcatgga ccagacgaac ccgctgtcgg gtctgaccca caagcgccgt 1320
   ctgaacgcgc tcggccccgg tggtctctcc cgtgagcggg cgggcttcga ggtccgtgac 1380
   gtgcacccgt cgcactacgg ccgcatgtgc ccgatcgaga cgcccgaagg cccgaacatc 1440
   ggtctgatcg gctcgctcgc ctcgtacggc cgggtcaacg cgttcggttt catcgagacc 1500
   ccgtaccgca aggtcgtcga cggtgtcgtc accgacgacg tcgactacct gacggccgat 1560
   gaagaggacc gcttcgtcat cgcgcaggcc aacgccccgc tcgcggacga cctgcgcttc 1620
   gccgagaacc gcgtcctggt ccgccgccgt ggcggcgagg tcgactacat ccccggcgac 1680
   gacgtcgact acatggacgt ctcaccgcgc cagatggtgt cggtcgcgac cgcgatgatc 1740
   cccttcctcg agcacgacga cgccaaccgc gcgctcatgg gctcgaacat gatgcgccag 1800
   gccgtgccgc tgatcaaggc ggagtccccg ctggtcggca ccggcatgga gtaccgctgt 1860
   gcggtcgacg ccggcgacgt catcaaggcc gagaaggacg gtgtcgtcca ggaggtctcc 1920
   gccgactacg tgacggtggc caacgacgac ggcacctaca ccacctaccg ggtggccaag 1980
   ttctcccgct ccaaccaggg cacctccttc aaccagaagg tcgtcgtgga cgagggtgcg 2040
   cgggtgatcg ccggccaggt gctggccgac ggcccgtcca ccgaggacgg cgagatggcg 2100
   ctcggcaaga acctcctggt ggcgttcatg ccgtgggagg gccacaacta cgaggacgcg 2160
   atcatcctca gccagcgtct ggtgcaggac gacgtcctct cctcgatcca catcgaggag 2220
   cacgaggtcg atgcccgtga caccaagctc ggccccgagg agatcacccg ggacatcccg 2280
   aacgtctccg aggaggtcct cgccgacctc gacgagcgcg gcatcatccg gatcggtgcc 2340
   gaggtcgtcg ccggcgacat cctggtcggc aaggtcaccc cgaagggcga gaccgagctg 2400
   accccggagg agcggctgct gcgcgcgatc ttcggtgaga aggcccgtga ggtccgtgac 2460
   acctcgctga aggtgccgca cggtgagatc ggcaaggtca tcggcgtccg cgtcttcgac 2520
   cgcgaagagg gcgacgaact gccgccgggc gtgaaccagc tggtccgcgt ctacgtggcg 2580
   cagaagcgca agatcaccga tggtgacaag ctcgccggcc gtcacggcaa caagggcgtc 2640
   atctccaaga tcctgccggt cgaggacatg ccgttcctgg aggacggcac cccggtcgac 2700
   atcatcctca acccgctggg tgtcccgtcc cgaatgaacc cgggacaggt cctggagatc 2760
   cacctgggct ggctggcctc ccgcggctgg aaggtcgagg gctccgagga ctggatgcag 2820
   cggctccagg ccatcggcgc cgacgaggtc gagcccggca ccaacgtcgc gaccccggtc 2880
   ttcgacggcg cccgcgagga cgagatcgcc ggtctcttcg actcgacgat cccgaaccgc 2940
   gacggcgacc gcctggtcca gtcgtccggc aaggcccggc tcttcgacgg ccgctccggc 3000
   gagccgttcc cggagccgat ctcggtcggc tacatgtaca tcctcaagct gcaccacctg 3060
   gtggacgaca agctgcacgc ccggtccacc ggtccgtact cgatgatcac ccagcagccg 3120
   ctgggtggta aggctcagtt cggtggccag cgcttcggtg agatggaggt gtgggcgctg 3180
   gaggcttatg gcgccgcgta cgccctccag gagctgctga ccatcaagtc cgacgacgtg 3240
   accggccgcg tgaaggtcta cgaggccatc gtcaagggcg agaacattcc cgagcccggc 3300
   atccccgagt ccttcaaggt gctcatcaag gagatgcagt ccctgtgcct caacgtggag 3360
   gtgctgtcgt ccgacggcat gtccatcgag atgcgcgaca ccgacgagga cgtcttccgc 3420
   gctgcggagg agctcggtat cgacctgtcc cggcgcgagc cgagcagcgt cgaagaggtc 3480
   tga 3483
<210> 9
   <211> 2031
   <212> DNA
   <213> Streptomyces
<220>
   <221> source
   <222> 1..2031
   <223> /mol_type="DNA" /note="gyr B" /organism="Streptomyces"
<400> 9
   gtgctgtgcc agaaagggcg cttcgtggcc gattccggca accccatcga aaacatcccg      60
   tccacgcccg acgacgaggc cctggctccg ccgtcgtacg acgccagtgc gattaccgtc     120
   ctggaagggc tggaggcggt ccgcaagcga cccggtatgt acatcggttc caccggtgag     180
   cgcggcctgc accatctcgt ccaagaggtc gtcgacaact ccgtcgacga ggccatggcc     240
   ggtcacgcgg acagcatcga ggtcacgatc ctcgccgacg gcggcgtccg cgtcgtggac     300
   aacggccgcg ggatcccggt gggcatcgtc ccctcggagg ggaagccggc tgtggaggtc     360
   gtgctgaccg tgctgcacgc gggcggcaag ttcggcggcg gcggctacgc cgtctccggc     420
   ggtctgcacg gcgtcggcgt ctccgtcgtc aacgccctgt cctcgaaggt gtcggtcgag     480
   gtcaagacgg acggctaccg ctggacccag gactacaaga cgggagcgcc gaccgcgccc     540
   ctggcccgga acgaggccac ggaggagacc ggcaccacgg tcaccttctg ggcggacccg     600
   gacgtcttcg agaccaccga gtactccttc gagacgctgg cccggcgctt ccaggagatg     660
   gcgttcctca acaagggcct gtcgatctcg ctcaaggacg agcgcgaggc ccatgtggac     720
   gaggagggca agccgctctc cgtgaagtac cactacgagg gcggcatcgt cgacttcgtg     780
   acctacctca actcccgcaa gggcgagctg gtccacccca cggtgatcgg gttcgaggcc     840
   gaggacaagg agcggatgct ctccctcgag atcgcgatgc agtggaacac ccagtacacc     900
   gagggtgtct acagcttcgc gaacaccatc cacacccatg agggcggcac ccacgaggag     960
   ggcttccgcg ccgcgctgac gtacctgatc aacaagtacg cgcgcgacaa gaagctgctc    1020
   cgggagcgtg acgacaacct caccggtgag gacatccgcg agggcctgac cgccatcatc    1080
   tcggtcaagc tgggcgagcc gcagttcgag ggccagacca agaccaagct gggcaacacg    1140
   gaggccaaga ccttcgtcca gaagatcgtc aacgagcatc tcgccgactg gctggaccgt    1200
   aaccctaatg aggcggcgga catcgtccgc aaggggatcc aggcggcgac ggcccgggtc    1260
   gcggcccgta aggcgcggga tctgacccgc cgtaaggggc tgctggagac cgcgtcgctg    1320
   ccgggcaagc tgagcgactg ccagtccaat gacccgtcga agtgcgagat cttcatcgtc    1380
   gagggtgact ccgccggcgg ctcggccaag tccggccgta acccgcagta tcaggcgatc    1440
   ctcccgatcc gcggcaagat cctcaacgtg gagaaggccc gggtcgacaa gatcctgcag    1500
   aacaacgagg tccaggcgct gatctccgcc ttcggcaccg gggtgcacga ggacttcgac    1560
   atcgccaagc tccgctatca caagatcatt ctgatggcgg acgccgatgt cgacggccag    1620
   cacatcaaca ccctgctgct gaccttcctc ttccgcttca tgcgcccgct ggtcgaggcg    1680
   gggcatgtct tcctctcccg tccgccgctc tacaagatca agtggggccg ggacgacttc    1740
   gagtacgcgt actcggaccg ggagcgggac gcgctgatcc aggtcggccg tgaacagggc    1800
   aagcgcatca gggacgactc ggtccagcgc ttcaagggtc tgggcgagat gaacgccgaa    1860
   gagctgcggg tcaccacgat ggaccccgac caccgcgtcc tgggccaggt caccctggac    1920
   gacgcggcgc aggccgacga cctgttctcg gtcctgatgg gtgaggacgt cgaggcacgg 1980
   cgctcgttca tccagcgcaa cgccaaggat gtccgcttcc tcgacatctg a 2031
<210> 10
   <211> 990
   <212> DNA
   <213> Streptomyces
<220>
   <221> source
   <222> 1..990
   <223> /mol_type="DNA" /note="Rec A" /organism="Streptomyces"
<400> 10
   ggcgatcggc cgaacgagcc ggtcgaggtc atccccaccg ggtcgaccgc tctcgacgtc 60
   gccctcggcg tcggcggtct gccgcgcggc cgggtggtcg aggtctacgg ccccgagtcc 120
   tccggtaaga cgaccctgac cctgcacgcg gtggccaatg cccagcgggc cggcggcacc 180
   gttgccttcg tggacgccga gcacgccctc gaccctgact acgcgcagaa gctgggcgtg 240
   gacaccgact ccctgatcct gtcccagccg gacaacggcg agcaggcgct cgagatcgtg 300
   gacatgctgg tccgctccgg cgccctcgac ctcatcgtca tcgactccgt cgccgccctg 360
   gtgccgcgcg cggagatcga gggcgagatg ggcgactccc acgtcggcct ccaggcccgg 420
   ctgatgagcc aggcgctccg taagatcacc agcgcgctca accagtccaa gaccaccgcg 480
   atcttcatca accagctccg cgagaagatc ggcgtgatgt tcggctcgcc ggagaccacg 540
   accggtggcc gggcgctgaa gttctacgcg tcggtgcgca tcgacatccg ccgcatcgag 600
   accctcaagg acggcaccga cgcggtcggc aaccgcaccc gcgtcaaggt cgtcaagaac 660
   aaggtcgcgc cgcccttcaa gcaggccgag ttcgacatcc tctacggcca gggcatcagc 720
   cgtgagggcg gtctgatcga catgggcgtc gagcacggct tcgtccgcaa gtccggtgcc 780
   tggtacacct acgagggcga ccagctcggc cagggcaagg agaacgcccg caacttcctg 840
   aaggacaacc ccgatctcgc caatgagatc gagaagaaga tcaaggaaaa gctcggcatc 900
   ggggtgaggc cccaggaccc ggcggccgcg gcacccacca cggacgcggc tggtgccgcg 960
   ggcgtgaccg acgccgcacc ggcgaaggcc 990
<210> 11
   <211> 1209
   <212> DNA
   <213> Streptomyces
<220>
   <221> source
   <222> 1..1209
   <223> /mol_type="DNA" /note="Trp B" /organism="Streptomyces"
<400> 11
   gtgcccagcg ccgagggcta tttcggcgcc ttcggcggca agttcatccc cgaggcgctc      60
   gtcgccgccg tcgacgaggt cgcggccgag tacgagaagg ccaagacgga ccccgccttc     120
   gcggccgagc tcgaggatct gctggtcaac tacaccggcc ggcccagtgc gctgaccgag     180
   gtgcggcggt tcgccgagca cgccgggggc gcccgggtct tcctcaagcg ggaggacctc     240
   aaccacaccg gctcccacaa gatcaacaat gtgctggggc aggccctgct caccaagcgc     300
   atgggcaagt cccgggtcat cgccgagacc ggcgccggtc agcacggcgt ggccacggcc     360
   accgcatgtg cgctgttcgg gctcgaatgc accatctaca tgggcgaggt cgacacccag     420
   cggcaggcgc tcaatgtggc gcggatgcgg atgctgggcg ccgaggtcat ctccgtgacc     480
   tccggcagcc gcaccctgaa ggacgccatc aacgaggcgt tccgggactg ggtcgccaat     540
   gtggaccgca cccactacct cttcggtacg gtggccggcc cccacccctt cccggcgctg     600
   gtgcgcgact tccaccgggt gatcggcgtg gaggcgcggc ggcagatcct ggagcggacc     660
   gggcggctgc cggacgcggt cgcggcctgt gtgggcggcg gatccaacgc gatcgggctg     720
   ttccacgcct tcctgccgga cgagagcgtg cgcctcgtcg gcttcgagcc cgccggacac     780
   ggtgtggaga ccggggagca cgcggccacg ctgagccagg gcgagcccgg gatcctgcac     840
   ggctcccggt cgttcgtgct ccaggacgag gacggccaga tcaccgagcc gtactcgatc     900
   tcggccggtc tcgactaccc cggcgtcggg ccggagcacg cgtatctgaa ggacatcggc     960
   cgtgccgagt accgggcggt caccgacgac gaggcgatgc gggcgctgcg gctgctctcg    1020
   gagaccgagg gcatcatccc ggcgatcgag agcgcccacg cgctggcggg cgccctggac    1080
   ctcggccgtg agctggggag cgacggcctg gtgctggtca acctctccgg gcgcggcgac    1140
   aaggacatgg acacggcggc tcggtacttc gggctctacg accagcagag cgaccaggga    1200
   gcgaagtga                                                            1209
<210> 12
   <211> 1419
   <212> DNA
   <213> Streptomyces
<220>
   <221> source
   <222> 1..1419
   <223> /mol_type="DNA" /note="Atp B" /organism="Streptomyces"
<400> 12
   gtggcctccg gccgcgtcgc gcgggtcatc ggcccggtcg tcgacgtgga gttccccgtc      60
   gacgcgatgc cggagatcta caacgcgctg caggtcgagg tcgccgaccc ctcccaggag     120
   ggggcgaaga agaccctgac cctcgaggtc gcccagcacc tcggcgaggg cctggtccgc     180
   gccatctcca tggagcccac cgacggcctg gtccgccagg ccgcggtgac cgacaccggc     240
   gacggcatca cggtgccggt cggcgatgtc accaagggcc gggtgttcaa caccctcggc     300
   aagatcctca acgagcccga ggccgagtcc gaggtcaccg agcgctggtc catccaccgc     360
   aaggccccgg ccttcgacca gctcgagtcc aagaccgaga tgttcgagac cggcctgaag     420
   gtcgtcgacc tgctgacccc gtacgtcaag ggcggcaaga tcggtctgtt cggcggcgcg     480
   ggcgtcggca agaccgtgct catccaggaa atgatcatgc gtgtggccaa gctgcacgag     540
   ggcgtttccg tgttcgccgg tgtcggcgag cgcacccgtg agggcaacga cctgatcgag     600
   gagatggccg agtccggcgt gctcccgcag accgcgctgg tcttcggcca gatggatgag     660
   cccccgggca cccgtctgcg cgtcgccctg gccggtctga ccatggcgga gtacttccgc     720
   gatgtgcaga agcaggacgt gctgttcttc atcgacaaca tcttccgctt cacccaggcc     780
   ggttccgagg tctcgaccct gctcggccgg atgccctccg cggtgggcta ccagccgaac     840
   ctggccgacg agatgggcat cctgcaggag cgcatcacct cgacccgtgg tcactcgatc     900
   acctcgatgc aggcgatcta cgtccccgcg gacgacctga ccgacccggc cccggcgacc     960
   accttcgcgc acctcgacgc gaccacggtg ctctcccggc cgatctcgga gaagggcatc    1020
   tacccggcgg tggacccgct ggactcgacg tcccggatcc tggacccgcg ctacatctcg    1080
   caggagcact acgactgcgc ctcgcgcgtg aagtcgatcc tgcagaagta caaggacctc    1140
   caggacatca tcaacatcct gggcatcgac gagctcggcg aggaggacaa gctcaccgtc    1200
   ttccgcgccc gccggatcga gcgcttcctg tcgcagaaca cccacgcggc gaagcagttc    1260
   accggcctcg acggatcgga tgtgccgctg gacgagtcca tcgccgcgtt caacgcgatc    1320
   gccgatggtg agttcgacca cttccccgag caggcgttct tcatgtgcgg tggcctggac    1380
   gacctcaagg ccaaggccaa ggagctgggc gtctcctga                            1419

## Description

L'invention concerne de façon générale les utilisations en agriculture d'une nouvelle bactérie du genre *Streptomyces.* L'invention concerne en particulier un procédé de traitement de végétaux, une composition de traitement de végétaux et les utilisations d'une telle composition de traitement pour le traitement de végétaux.

Le développement de cultures végétales agricoles intensives est aujourd'hui nécessaire pour satisfaire les besoins alimentaires dans le monde. À cet effet, de telles cultures végétales agricoles intensives nécessitent l'utilisation d'agents visant à optimiser la croissance des végétaux d'intérêt et à minimiser la croissance de végétaux et/ou d'agents pathogènes indésirables. En particulier, on connaît des agents visant à améliorer la nutrition des végétaux, et des agents de protection des végétaux vis-à-vis d'organismes pathogènes, notamment vis-à-vis de bactéries phyto-pathogènes et/ou de champignons qui sont susceptibles d'affecter la croissance optimale des végétaux.

On connaît aussi des produits de fertilisation des sols. Il s'agit en général d'engrais issus de l'industrie chimique, qui se présentent sous une forme solide et peu soluble dans le sol et qui nécessitent par conséquent d'être apportés massivement au moment précis où ces produits de fertilisation sont nécessaires pour la croissance optimale des végétaux. L'excès de ces produits de fertilisation solides apportés au sol et non prélevés par les végétaux reste momentanément dans le sol et est dissout progressivement par les eaux de pluie ou les eaux d'arrosage avant d'atteindre les nappes phréatiques et les polluer.

Des solutions pour améliorer la fertilisation des sols sans polluer l'environnement et en particulier les nappes phréatiques tout en favorisant la nutrition et la croissance des végétaux sont donc recherchées.

L'invention vise donc à apporter une solution à ce problème.

Par ailleurs, de nombreux agents phyto-pathogènes sont susceptibles de se développer dans les cultures végétales et à leurs dépens en diminuant leur rendement de production et la qualité de la production végétale.

L'invention vise donc aussi à apporter une solution pour la protection des végétaux vis-à-vis de certains organismes -bactéries et/ou champignons- phyto-pathogènes.

Pour ce faire, l'invention concerne un procédé de traitement d'une matière végétale, selon la revendication 1.

La séquence SEQ ID_NO1 est décrite ci-après :

Dans la séquence SEQ ID_NO1 ci-dessus, le symbole « n » en positions 1036 et 1049 de la séquence SEQ ID_NO1 désigne, selon l'IUPAC (« *International Union of Pure and Applied Chemistry* »), l'un quelconque des quatre nucléotides a, t, c ou g. Ainsi, le nucléotide n en position 1036 est choisi dans le groupe formé du nucléotide « a », du nucléotide « t », du nucléotide « g » et du nucléotide « c » et le nucléotide n en position 1049 est choisi, indépendamment du nucléotide en position 1036, dans le groupe formé du nucléotide « a », du nucléotide « t », du nucléotide « g » et du nucléotide « c ».

L'invention concerne donc un procédé de traitement d'une matière végétale, dans lequel on applique la composition de traitement sur au moins une partie de ladite matière végétale ou sur un substrat de culture de ladite matière végétale.

Les inventeurs ont observé que, de façon totalement surprenante et imprévisible, une composition de traitement selon l'invention présente des capacités inhibitrices de la croissance de certaines bactéries telles que, par exemple, *Micrococcus luteus, Bacillus subtilis* et de certaines bactéries ou champignons phyto-pathogènes telles que *Streptomyces scabies, Botrytis cinerae, Fusarium culmorum, Pythium ultimum, Phaeomoniella chlamydospora*, *Phaeomoniella aelophilum, Eutypa lata*, *Fomitiporia mediterranea* et *Botryosphaeria obtusa*.

Une souche de bactéries présentant une séquence d'ADN homologue à 100 % avec la SEQ ID_NO1 a été déposée par le demandeur et enregistrée en date du 7 avril 2011 sous le numéro I-4467 auprès de la Collection Nationale de Cultures de Microorganismes (CNCM) de l'Institut Pasteur (dont l'adresse est 25, rue du Docteur Roux, 75724 Paris cedex 15) ayant le statut d'autorité de dépôt internationale selon le traité de Budapest.

Dans un procédé de traitement décrit ici, on utilise des bactéries qui sont du genre *Streptomyces* et qui présentent une séquence d'ADN homologue à 100 % avec la SEQ ID_NO1.

Avantageusement et selon l'invention, la bactérie isolée présente une séquence d'ADN codant pour la sous unité béta de l'ARN polymérase (polB) homologue avec la SEQ ID_NO8. La bactérie isolée présente donc la séquence SEQ ID_NO8 à titre de séquence codant pour la sous unité béta de l'ARN polymérase (polB).

Avantageusement et selon l'invention, la bactérie isolée présente une séquence d'ADN codant pour la gyrase (gyrB) homologue avec la SEQ ID_NO9. La bactérie isolée présente donc la séquence SEQ ID_NO9 à titre de séquence codant pour la gyrase (gyrB).

Avantageusement et selon l'invention, la bactérie isolée présente une séquence d'ADN codant pour la recombinase (RecA) homologue avec la SEQ ID_NO10. La bactérie isolée présente donc la séquence SEQ ID_NO10 à titre de séquence codant pour la recombinase (RecA).

Avantageusement et selon l'invention, la bactérie isolée présente une séquence d'ADN codant pour la sous unité béta de la tryptophane synthase (trpB) homologue avec la SEQ ID_NO11. La bactérie isolée présente donc la séquence SEQ ID_NO11 à titre de séquence codant pour la tryptophane synthase (trpB).

Avantageusement et selon l'invention, la bactérie isolée présente une séquence d'ADN codant pour la sous unité béta de l'ATP synthase (AtpB) homologue avec la SEQ ID_NO12. La bactérie isolée présente donc la séquence SEQ ID_NO12 à titre de séquence codant pour la sous unité béta de l'ATP synthase (AtpB).

Avantageusement et selon l'invention, la bactérie isolée présente au moins l'une des séquences SEQ ID_NO4, SEQ ID_NO5, SEQ ID_NO6, SEQ ID_NO7 et SEQ ID_NO8. Avantageusement et selon l'invention, la bactérie isolée présente chacune des séquences SEQ ID_NO4, SEQ ID_NO5, SEQ ID_NO6, SEQ ID_NO7 et SEQ ID_NO8.

Avantageusement et selon l'invention, la composition de traitement comprend des bactéries choisies dans le groupe formé des bactéries conformes à la souche déposée et enregistrée en date du 7 avril 2011 sous le numéro I-4467 auprès de la Collection Nationale de Cultures de Microorganismes (CNCM) de l'Institut Pasteur et des bactéries mutantes de cette souche déposée obtenues par mutagenèse dirigée et comprenant une partie de séquence homologue à 100% avec la séquence SEQ ID_NO1.

Dans une première variante d'un procédé selon l'invention, on utilise des bactéries conformes à la souche déposée et enregistrée en date du 7 avril 2011 sous le numéro I-4467 auprès de la Collection Nationale de Cultures de Microorganismes (CNCM) de l'Institut Pasteur, de telles bactéries conformes à la souche déposée comprenant une partie de séquence d'ADN présentant 100 % d'homologie avec la séquence SEQ ID_NO1.

La souche déposée auprès de la CNCM sous le n° I-4467 forme, lorsqu'elle est cultivée sur milieu ISP-2 ou sur milieu Bennett solide, des amas ou « colonies » bactériennes présente :
- un mycélium, dit mycélium de substrat, ramifié se développant dans l'épaisseur du milieu solide et de couleur variant du jaune-brun au gris-brun selon la composition du milieu solide ;
- un mycélium aérien se développant à l'interface air/solide qui est de couleur blanche.

La souche déposée à la CNCM est une souche de bactéries isolées à partir de tout milieu naturel. En particulier, la souche déposée à la CNCM a été isolée à partir d'un milieu naturel d'origine dans lequel est préexistait.

Les bactéries de la souche déposée à la CNCM sont caractérisées par tout ou partie des caractéristiques ci-après :
- elles sont non pathogènes (inoffensives) pour l'homme ;
- ce sont des bactéries à Gram positif ;
- ce sont des bactéries saprophytes, c'est-à-dire capables de dégrader la matière organique du sol ;
- elles présentent une température optimale de croissance qui est comprise entre 12°C et 37°C, préférentiellement entre 28°C et 30°C ;
- elles présentent un pH optimal de croissance compris entre 6 et 8 ;
- elles sont aptes à utiliser le D-glucose, le mannitol, le lactose, le saccharose, le maltose et la dextrine à titre de source de carbone ;
- elles ne sont pas aptes à utiliser préférentiellement le galactose, l'inositol, le sorbose, le fructose, l'arabinose, le raffinose, le rhamnose la cellulose à titre de source unique de carbone ;
- elles sont aptes à utiliser les acides aminés, les sels de nitrate et les sels d'ammonium à titre de source d'azote ;
- elles sont aptes à réduire les nitrates en nitrites, à dégrader l'adénine, le tweed 20 et l'acétate de sodium ;
- elles ne sont pas aptes à hydrolyser l'amidon et à utiliser ces produits d'hydrolyse.

Dans une deuxième variante d'un procédé selon l'invention, on utilise des bactéries mutantes des bactéries de la souche déposée à la CNCM, c'est-à-dire obtenues par mutation des bactéries de la souche déposée à la CNCM, de telles bactéries mutantes comprenant une séquence d'ADNr 16 Shomologue à 100 % avec la séquence SEQ ID_NO1.

On obtient de telles bactéries mutantes par traitement de bactéries de la souche déposée à la CNCM par toute méthode de mutagenèse, notamment choisie dans le groupe formé des méthodes de mutagenèse aléatoire et des méthodes de mutagenèse dirigée.

On réalise un traitement par mutagenèse aléatoire dans lequel on soumet des bactéries conformes à la souche déposée à la CNCM à au moins un agent mutagène choisi dans le groupe formé des agents mutagènes physiques -notamment en exposant des bactéries conformes à la souche déposée à la CNCM à un rayonnement lumineux ultraviolet ou à une radiation ionisante-et des agents mutagènes chimiques.

Des bactéries mutantes présentent des différences de séquence d'ADN par rapport aux bactéries de la souche déposée à la CNCM. Ces différences de séquence d'ADN affectent des séquences d'ADN distinctes de la séquence de l'ADNᵣ 16S de la bactérie selon l'invention.

Selon l'une des première ou deuxième variantes avantageuses d'un procédé selon l'invention, on met la composition de traitement comprenant des bactéries présentant une séquence d'ADN homologue à 100 % avec la SEQ ID_NO1 (c'est-à-dire des bactéries conformes à la souche déposée à la CNCM et/ou des bactéries mutantes de cette souche déposée) en contact avec au moins une partie de ladite matière végétale.

Selon ces première ou deuxième variantes d'un procédé selon l'invention, les bactéries de la composition de traitement peuvent être des bactéries en phase de croissance végétative. On entend par « bactéries en phase de croissance végétative », des bactéries présentant un métabolisme actif et/ou se multipliant par division cellulaire. Il s'agit donc de bactéries vivantes en phase de croissance végétative ou en phase stationnaire. La composition de traitement est alors formée de bactéries et d'un milieu de culture. Le milieu de culture est avantageusement un milieu de culture aqueux. Avantageusement, le milieu de culture aqueux est un milieu de culture choisi dans le groupe formé des milieux riches comprenant tous les éléments minéraux et des précurseurs organiques nécessaires à la croissance des bactéries selon l'invention, en particulier une source de carbone, une source d'azote, une source de phosphore, des vitamines et des oligoéléments.

Avantageusement, le milieu de culture aqueux peut comprendre du D-glucose, un extrait de levure, du phosphate de potassium dibasique, du sulfate d'ammonium, du chlorure de potassium et du glycérol.

Selon ces première ou deuxième variantes d'un procédé selon l'invention, les bactéries de la composition de traitement peuvent aussi être sous forme de spores présentant la séquence d'ADN homologue à 100 % avec la séquence SEQ ID_NO1. On observe la formation de spores lorsque les bactéries en phase de croissance végétative forment un mycélium primaire se développant à une interface air/solide et initient une croissance aérienne. Des filaments ou « hyphes » aériens non ramifiés se développent à partir du mycélium primaire et présentent à leurs extrémités des structures compartimentées précurseurs de spores. Il peut s'agir de spores de bactéries conformes à la souche déposée et enregistrée en date du 7 avril 2011 sous le numéro I-4467 auprès de la Collection Nationale de Cultures de Microorganismes (CNCM) de l'Institut Pasteur ou de bactéries mutantes de cette souche déposée et comprenant une séquence d'ADN présentant la séquence SEQ ID_NO1. Il s'agit donc de bactéries vivantes sous forme de spores.

Les spores de bactéries de la souche déposée à la CNCM sont des spores lisses et enchaînées en spirales de type « S », les chaînes de spores lisses présentant en moyenne de 10 à 50 spores.

On favorise l'obtention de spores de la bactérie selon l'invention par culture de bactéries selon l'invention dans un milieu de culture susceptible de générer un stress bactérien, notamment un milieu de culture liquide limité ou carencé en carbone et/ou en azote et/ou en phosphore et/ou en vitamines et/ou en oligoéléments.

Avantageusement, de telles spores sont susceptibles d'être placées dans un milieu riche et de réhydratation pour former une population de bactéries selon l'invention en phase végétative.

Avantageusement, on applique sur la matière végétale une composition de traitement comprenant des bactéries en phase végétative et/ou des bactéries sous forme de spores. En tout état de cause, les formes végétatives et les spores des bactéries présentent une séquence d'ADNᵣ 16S homologue à 100 % avec la séquence SEQ ID_NO1.

Le milieu de culture aqueux peut être un milieu de culture aqueux solide ou un milieu de culture aqueux liquide.

Dans un première mode de réalisation de cette première variante d'un procédé selon l'invention, la composition de traitement comprenant des bactéries (souche déposée et/ou bactéries mutantes de la souche déposée) présentant la séquence d'ADNr 16S conforme à la SEQ ID_NO1 est une composition de traitement liquide. Avantageusement, la composition de traitement liquide est une composition de traitement liquide aqueuse. La composition de traitement liquide aqueuse peut donc être formée d'une charge bactérienne comprenant des bactéries présentant la séquence d'ADNr 16S conforme à la SEQ ID_NO1 dans un milieu liquide aqueux.

Dans ce premier mode de réalisation, avantageusement, on applique sur au moins une partie de ladite matière végétale la composition de traitement liquide comprenant des bactéries de la souche déposée à la CNCM et/ou des bactéries mutantes dans un milieu de culture liquide.

Dans un deuxième mode de réalisation de cette première variante d'un procédé selon l'invention, la composition de traitement comprenant les bactéries présentant la séquence SEQ ID_NO1 est une composition de traitement solide.

Dans ce deuxième mode de réalisation, avantageusement, on applique sur au moins une partie de ladite matière végétale la composition de traitement solide comprenant des bactéries de la souche déposée à la CNCM et/ou des bactéries mutantes et un milieu de culture solide. Dans ce deuxième mode de réalisation, le milieu de culture solide peut comprendre une proportion d'agar-agar (E406).

Dans une troisième variante d'un procédé selon l'invention, on applique une composition de traitement comprenant au moins un polynucléotide présentant au moins une partie de séquence d'ADN homologue à la SEQ ID_NO1.

Avantageusement, dans cette troisième variante d'un procédé selon l'invention, la composition de traitement peut comprendre un tel polynucléotide et/ou des bactéries choisies dans le groupe formé des bactéries conformes à la souche déposée et enregistrée en date du 7 avril 2011 sous le numéro I-4467 auprès de la Collection Nationale de Cultures de Microorganismes (CNCM) de l'Institut Pasteur et/ou des bactéries mutantes de cette souche déposée.

Avantageusement, dans cette troisième variante d'un procédé selon l'invention, la composition de traitement peut être une composition acellulaire, c'est à dire sensiblement exempte de bactéries présentant la séquence SEQ ID_NO1. Dans cette troisième variante d'un procédé selon l'invention, on met en contact au moins une partie de ladite matière végétale avec la composition acellulaire débarrassée des bactéries présentant la séquence SEQ ID_NO1.

Avantageusement et selon l'invention, une telle composition acellulaire est en particulier un milieu de culture dans lequel se sont développées des bactéries comprenant la séquence SEQ ID_NO1 et qui est sensiblement exempt desdites bactéries.

L'invention vise aussi une composition de traitement formée d'un milieu de culture bactérien présentant un chromatogramme HPLC comprenant un premier signal majeur (P_{72,6}) à un temps de rétention de 11,925 min et un deuxième signal majeur (P_{72,14}) à un temps de rétention de 20,04 min.

Une telle composition de traitement formée d'un milieu de culture bactérien selon l'invention présente un effet stimulateur de la croissance de plantes en culture telles que le tournesol, le maïs, le colza, le blé et la tomate et présente aussi une activité antifongique -notamment vis-à-vis de *Botrytis cinerea-* sur les feuilles de vigne.

Une telle composition de traitement peut être un milieu de culture dans lequel se sont développées des bactéries comprenant la séquence SEQ ID_NO1 et qui est sensiblement exempt ou non desdites bactéries.

Une composition acellulaire selon l'invention obtenue après 24 heures de culture de la souche selon l'invention peut présenter par analyse en HPLC sur colonne en phase inverse une pluralité de pics (P_{24,i}) présentant des temps de rétention (tᵢ) listés dans le tableau 1 ci-après.

**Tableau 1**

| P₂₄,ᵢ | tᵢ (minute) |
|---|---|
| P₂₄,₁ | 3,30 - 3,60 |
| P₂₄,₂ | 3,90 - 4,0 |
| P₂₄,₃ | 5,90 - 6,38 |
| P₂₄,₄ | 10,1 - 10,20 |
| P₂₄,₅ | 13,5 - 13,9 |
| P₂₄,₆ | 21,4 - 21,7 |

Une telle composition acellulaire selon l'invention obtenue après 3 jours (72 heures) de culture de la souche selon l'invention peut présenter en analyse par HPLC sur colonne en phase inverse une pluralité de pics (P_{72,i}) présentant des temps de rétention (tᵢ) et des aires (A_{72,i}) exprimées en valeurs relatives sous lesdits pics (P_{72,i}) listés dans le tableau 2 ci-après.

**Tableau 2**

| P_{72,i} | tᵢ (minute) | A_{72,i} (%) |
|---|---|---|
| P_{72,1} | 3,93 | 3,45 |
| P_{72,2} | 5,30 | 2,02 |
| P_{72,3} | 6,59 | 5,05 |
| P_{72,4} | 8,83 | 1,45 |
| P₇₂,₅ | 9,63 | 3,13 |
| P₇₂,₆ | 11,925 | 9,07 |
| P₇₂,₇ | 12,39 | 1,92 |
| P₇₂,₈ | 12,87 | 1,24 |
| P₇₂,₉ | 13,76 | 1,82 |
| P₇₂,₁₀ | 15,93 | 2,68 |
| P₇₂,₁₁ | 16,27 | 2,07 |
| P₇₂,₁₂ | 18,41 | 1,98 |
| P₇₂,₁₃ | 19,38 | 3,15 |
| P₇₂,₁₄ | 20,04 | 6,59 |

On analyse par HPLC l'extrait obtenu par extraction du milieu de culture de la souche selon l'invention par l'acétate d'éthyle, séchage de la solution d'acétate d'éthyle et solubilisation de l'extrait dans le méthanol. La chromatographie HPLC est réalisée sur une colonne Xbridge (Waters, Guyancourt, France) de dimensions 25cm/4,6mm/5µm. L'élution est réalisée par un gradient d'acétonitrile de 20 % à 95 % dans l'eau avec un débit de 0,8 mL/min. La détection est réalisée à la longueur d'onde de 254 nm.

Dans les conditions d'extraction et d'analyse ci-dessus mentionnées, avantageusement et selon l'invention, la composition de traitement est une composition acellulaire sensiblement exempte de bactéries présentant la séquence SEQ ID_NO1 et formée d'un milieu de culture dans lequel se sont développées des bactéries comprenant ladite séquence SEQ ID_NO1 et qui est sensiblement exempt desdites bactéries et qui présente un chromatogramme HPLC comprenant un premier signal majeur (P_{72,6}) à un temps de rétention de 11,925 min et un deuxième signal majeur (P_{72,14}) à un temps de rétention de 20,04 min.

Dans les conditions d'analyse ci-dessus mentionnées, avantageusement et selon l'invention, la composition acellulaire obtenue après 3 jours (72 heures) de culture de la souche selon l'invention présente un chromatogramme HPLC comprenant des signaux, dits signaux mineurs, à des temps de rétention de 3,93 min, de 5,30 min, de 6,59 min, de 8,83 min, de 9,63 min, de 12,39 min, de 12,87 min, de 13,76 min, de 13,76 min, de 15,93 min, de 16,27 min, de 18,41 min et de 19,38 min.

Une telle composition acellulaire est susceptible de pouvoir être obtenue par un procédé dans lequel :
- on met en culture au moins une bactérie de la souche déposée à la CNCM et présentant la séquence d'ADN SEQ ID_NO1 dans le milieu de culture apte à permettre la croissance desdites bactéries pendant une durée supérieure à 24 heures, puis ;
- on élimine les bactéries du milieu de culture de façon à former la composition acellulaire au moins sensiblement exempte desdites bactéries.

Avantageusement, on ensemence et on met en culture les bactéries dans le milieu de culture et pendant une durée suffisante aptes à permettre leur croissance, c'est-à-dire pendant une durée comprise entre une durée minimale de l'ordre de 24 heures et 10 jours, à une température comprise entre 12°C et 37°C, préférentiellement comprise entre 28°C et 30°C, en particulier de l'ordre de 30°C, puis on extrait les bactéries du milieu de culture -par exemple par centrifugation du milieu de culture- ou on inactive les bactéries de façon à former la composition acellulaire. Une telle composition acellulaire est donc un milieu de culture qui ne comprend plus les bactéries ou qui comprend des bactéries mortes, qui a été obtenue par mise en contact et culture de bactéries dans un milieu de culture et dans des conditions adaptés à la croissance desdites bactéries.

Il est possible d'obtenir une inactivation des bactéries pour toute technique connue, notamment par lyse des bactéries par traitement enzymatique ou par traitement mécano-chimique.

Avantageusement et selon l'invention, on applique la composition de traitement sur la(les) partie(s) de la matière végétale, ladite composition de traitement comprenant en outre au moins un excipient acceptable (phyto-acceptable) pour ladite matière végétale.

Avantageusement et selon l'invention, on choisit la matière végétale dans le groupe formé de tout ou partie d'un végétal en culture, d'un fruit ou d'un légume après récolte, de semences -notamment de graines, de bulbes, de griffes, de rhizomes ou de tubercules- de végétaux. En particulier, on applique la composition de traitement sur au moins une partie aérienne de la plante, par exemple sur le feuillage ou sur les graines.

Avantageusement et selon l'invention, on choisit le végétal en culture dans le groupe formé des arbres fruitiers -notamment des oliviers, des abricotiers, des cerisiers, des cognassiers, des amandiers, des figuiers, des noisetiers, des noyers, des pêchers, des poiriers, des pommiers, des pruniers, de la vigne et des agrumes-, des arbres et arbustes d'ornementation, des plantes potagères -notamment des asperges, des aubergines, des blettes, des betteraves potagères, des carottes, du céleri, de la chicorée, des endives, des crucifères ou brassicacées (par exemple les choux), des concombres, des cornichons, des courgettes, des échalotes, des oignons, des fèveroles, des féveroles de printemps, des féveroles d'hiver, des fraisiers, des framboisiers, des haricots, des laitues, des chicorées frisées, des chicorées scaroles, du houblon, des lentilles, de la luzerne, de la mâche, du maïs, des melons, des navets, des poireaux, des pois, des poivrons, des pommes de terre, des radis, des rutabagas, du soja, du tabac, des tomates, des tournesols-, des céréales -notamment du blé, du colza, du lin, du lin oléagineux, du lin textile, de l'orge, du sorgho-, des cultures florales diverses -notamment des chrysanthèmes, des hortensias, des oeillets, des rosiers, des tulipes- et des plantes aromatiques -notamment du persil, de l'ail, de la ciboulette-.

Avantageusement et selon l'invention, la matière végétale est choisie dans le groupe formé des semences de végétaux, des parties aériennes de végétaux et des racines de végétaux.

Avantageusement, on applique la composition de traitement sur des semences de végétaux pour activer leur germination.

Avantageusement, on applique la composition de traitement sur les parties aériennes des végétaux pour un traitement phytosanitaire des végétaux ou pour un traitement de stimulation des défenses naturelles des végétaux ou pour un traitement de stimulation de la croissance des végétaux.

L'invention vise donc un procédé de traitement d'une matière végétale dans lequel on applique sur au moins une partie de ladite matière végétale une composition de traitement comprenant au moins une matière biologique choisie dans le groupe formé :
- d'au moins une bactérie comprenant une séquence d'ADN homologue à 100 % avec la SEQ ID_NO1 ;
- d'une composition liquide comprenant une séquence d'ADN homologue à 100 % avec la SEQ ID_NO1 ;
- d'une composition solide comprenant une séquence d'ADN homologue à 100 % avec la SEQ ID_NO1 ;
- d'une composition acellulaire comprenant une séquence d'ADN homologue à 100 % avec la SEQ ID_NO1.

### Nutrition

Avantageusement et selon l'invention, on utilise une composition de traitement comprenant un nutriment solide à l'état divisé. On applique donc la composition de traitement comprenant en outre au moins un nutriment solide à l'état divisé sur tout ou partie du végétal en culture. La composition de traitement comprenant au moins un nutriment à l'état solide et au moins un agent biologique comprenant une séquence d'ADN homologue à plus de 99 % -borne exclue- avec la SEQ ID_NO1. En particulier, avantageusement et selon l'invention, on applique ladite composition de traitement sur le substrat -notamment sur le sol- de culture du végétal. Cependant, il est aussi possible d'appliquer la composition de traitement sur les parties aériennes du végétal. Les inventeurs ont observé qu'une composition de traitement comprenant des bactéries comprenant une séquence d'ADN homologue à plus de 99 % -borne exclue- avec la SEQ ID_NO1 -notamment des bactérie de la souche telle que déposée à la CNCM- permet d'améliorer la solubilisation de nutriments solides -notamment du phosphore- d'un substrat de culture de végétaux, favorisant ainsi la nutrition et la fertilisation des végétaux.

### Stimulation de la croissance de végétaux

Avantageusement et selon l'invention, on applique ladite composition de traitement sur ladite matière végétale pour activer la croissance de cette dernière.

En particulier, avantageusement et selon l'invention, on applique ladite composition de traitement sur des graines, de façon à activer la germination des graines. Les inventeurs ont observé que l'application d'une composition de traitement selon l'invention comprenant des bactéries à l'état végétatif et/ou sous forme de spores sur graines -notamment sur des graines de tournesol ou sur des graines de maïs- permet d'accélérer la germination des graines et croissance des plants de tournesol et de maïs.

### Inhibition de phyto-pathogènes

Avantageusement et selon l'invention, on applique ladite composition de traitement sur ladite matière végétale pour inhiber la croissance d'au moins un microorganisme cible. On applique ladite composition de traitement sur ladite matière végétale pour inhiber la croissance de microorganismes cibles choisis dans le groupe formé des microorganismes (bactéries ou champignons) phyto-pathogènes. En particulier, avantageusement et selon l'invention, on applique ladite composition de traitement sur au moins une partie de feuillage de végétaux. Il est possible que la composition de traitement soit une composition de traitement curatif ou une composition de traitement prophylactique (ou préventif) de ladite matière végétale. Dès lors on applique la composition de traitement préalablement à l'apparition de la maladie ou après l'apparition de la maladie.

### Stimulation des défenses naturelles des plantes

Avantageusement et selon l'invention, on applique ladite composition de traitement sur ladite matière végétale pour stimuler les défenses naturelles de ladite matière végétale.

Les inventeurs ont observé de façon imprévisible et inattendue qu'une composition de traitement selon l'invention permet de stimuler les mécanismes d'autodéfense des végétaux -notamment d'activer les flux ioniques et/ou l'expression du gène PR-1 (« Pathogenesis-Related protein of type 1 »)- responsable de la synthèse de composés de défense chez la plante.

L'invention s'étend à une composition de traitement d'une matière végétale comprenant au moins un agent biologique choisi dans le groupe formé :
- des bactéries comprenant une séquence d'ADN, dit ADNᵣ 16S, codant pour l'ARN ribosomal 16S de ladite bactérie homologue à 100 % avec la SEQ ID_NO1,
- des milieux de culture dans lesquels se sont développées des bactéries comprenant la séquence d'ADNr 16S homologue à 100 % avec la SEQ ID_NO1 et qui sont sensiblement exempts desdites bactéries, lesdits milieux de culture comprenant des polynucléotides présentant une séquence d'ADN homologue à 100 % avec la SEQ ID_NO1, et dans lequel les bactéries sont choisies dans le groupe formé des bactéries conformes à la souche déposée et enregistrée en date du 7 avril 2011 sous le numéro I-4467 auprès de la Collection Nationale de Cultures de Microorganismes (CNCM) de l'Institut Pasteur, et des bactéries mutantes de cette souche déposée.

L'invention s'étend à une telle composition de traitement d'une matière végétale.

Les inventeurs ont observé qu'une telle composition de traitement selon l'invention :
- est apte à ralentir la croissance la croissance de certaines bactéries telles que *Micrococcus luteus* et *Bacillus subtilis ;*
- est apte à ralentir la croissance de certains micro-organismes cible phyto-pathogènes tels que *Botrytis cinerea*, *Streptomyces scabies, Botrytis cinerae, Fusarium culmorum, Pythium ultimum, Phaeomoniella chlamydospora*, *Phaeomoniella aelophilum, Eutypa lata*, *Fomitiporia mediterranea* et *Botryosphaeria obtusa*, et ;
- présente un effet stimulateur de la croissance de plantes en culture telles que le tournesol et le maïs, et ;
- est apte à stimuler les défenses naturelles de végétaux en culture.

En particulier, les inventeurs ont découvert qu'une composition de traitement selon l'invention présente une activité stimulatrice des défenses naturelles des plantes, c'est-à-dire sont aptes à activer l'expression chez les plantes de gènes de défense -par exemple PR-1- vis-à-vis d'organismes pathogènes des plantes.

Avantageusement et selon l'invention, la composition de traitement est liquide. En particulier, la composition de traitement est liquide à température ambiante.

Avantageusement et selon l'invention, la composition de traitement est solide. En particulier, la composition de traitement est solide à température ambiante.

Avantageusement et selon l'invention, la composition de traitement comprend au moins un excipient acceptable (phyto-acceptable) pour permettre son application sur une matière végétale à traiter.

L'invention vise aussi toute utilisation en agriculture d'une composition de traitement selon l'invention, c'est-à-dire d'une composition de traitement comprenant au moins un agent biologique choisi dans le groupe formé :
- des bactéries comprenant une séquence d'ADN, dit ADNᵣ 16S, codant pour l'ARN ribosomal 16S de ladite bactérie homologue à 100 % avec la SEQ ID_NO1,
- des polynucléotides présentant une séquence d'ADN homologue à 100 % avec la SEQ ID_NO1,
et dans lequel les bactéries sont choisies dans le groupe formé des bactéries conformes à la souche déposée et enregistrée en date du 7 avril 2011 sous le numéro I-4467 auprès de la Collection Nationale de Cultures de Microorganismes (CNCM) de l'Institut Pasteur, et des bactéries mutantes de cette souche déposée.

En particulier, l'invention vise toute utilisation en agriculture d'une composition de traitement selon l'invention comprenant au moins une bactérie de la souche telle que déposée à la CNCM et présentant la séquence d'ADN SEQ ID_NO1.

Plus particulièrement, l'invention vise l'utilisation d'une telle composition de traitement
pour :
- stimuler la croissance de végétaux, ou pour ;
- stimuler la germination de graines, ou pour ;
- un traitement de fertilisation de végétaux, ou pour ;
- un traitement antimicrobien -notamment un traitement antifongique ou un traitement antibactérien- et/ou antiviral de végétaux, ou pour ;
- stimuler les défenses naturelles des végétaux.

L'invention concerne également un procédé de traitement d'une matière végétale, une composition de traitement d'une matière végétale et l'utilisation de telles compositions de traitement en agriculture caractérisés en combinaison par tout ou partie des caractéristiques mentionnées ci-dessus ou ci-après.

D'autres buts, caractéristiques et avantages de l'invention apparaîtront à la lecture de la description suivante, des exemples illustratifs donnés uniquement à titre non limitatif et des figures annexées dans lesquelles :
- la figure 1 est une reproduction de photographies comparatives (la et 1b) de feuilles de vigne montrant l'effet protecteur/curatif vis-à-vis de *Botrytis cinerae* d'un traitement avec une composition de traitement selon l'invention ;
- la figure 2 est une reproduction d'une photographie montrant l'inhibition de la croissance d'un champignon pathogène par les bactéries selon l'invention ;
- la figure 3 est une reproduction d'une photographie illustrative de la solubilisation du phosphate de calcium par des bactéries selon l'invention ;
- la figure 4 est une reproduction d'une photographie illustrative de la stimulation de la croissance d'un plant de tournesol par traitement des graines de tournesol avec une composition liquide selon l'invention ;
- la figure 5 est une reproduction d'une photographie illustrative de la stimulation de la croissance d'un plant de maïs par traitement des graines de maïs avec une composition liquide selon l'invention ;

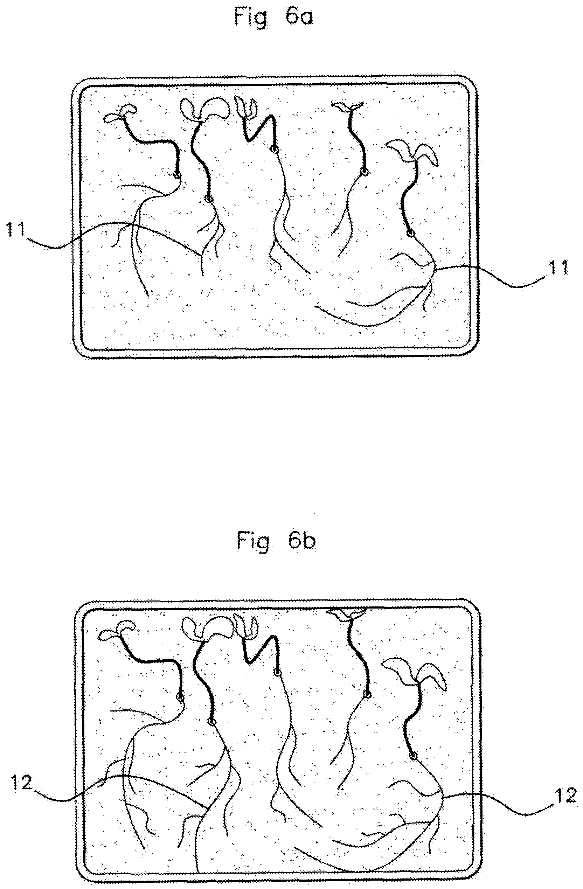

- les figures 6a et 6b sont des reproductions de photographies illustratives de la stimulation de la croissance racinaire de plantules de colza par une composition liquide comprenant des bactéries selon l'invention ;
- la figure 7 est une photographie d'un gel d'électrophorèse montrant la stimulation de l'expression de la protéine PR-1 de défense naturelle des plantes par une composition selon l'invention ;
- la figure 8 est une représentation graphique de l'effet d'une composition selon l'invention sur la stimulation du flux calcique, et ;
- la figure 9 est une représentation graphique de l'effet d'une composition selon l'invention sur l'expression du gène PR-1 d'une plante.

La souche de *Streptomyces* déposée à la CNCM sous le n° I-4467 a été isolée à partir d'un échantillon de la rhizosphère et des racines profondes d'un cep de vigne. Cet échantillon de la rhizosphère prélevé à une profondeur comprise entre 10 cm et 50 cm sous la surface du sol en éliminant la partie superficielle du prélèvement, a été placé dans un sachet stérile fermé hermétiquement et conservé à + 4°C préalablement à l'isolement des souches de *Streptomyces.*

L'échantillon prélevé est mis en suspension dans de l'eau distillée stérile à raison de 4 g d'échantillon dans 36 mL d'eau sous agitation magnétique à une vitesse de 200 rotations par minutes pendant 30 minutes. La suspension obtenue est ensuite placée à la température de 50°C pendant 10 min. La suspension est ensuite diluée dans l'eau distillée stérile selon un facteur de dilution de 10⁻⁷. 0,1 mL de cette dilution sont étalés stérilement sur boîte de pétri comprenant un milieu de culture gélosé solide SEA (« Soil Extract Agar ») complémenter avec de l'acide nalixidique (10 mg/L) ou de la novobiocine (25 mg/L) à titre d'antibiotiques et/ou de cycloheximide (40 mg/L) à titre d'antifongique. Les boîtes de pétri sont ensuite placées à la température de 30°C dans un incubateur pendant 21 jours.

On isole les bactéries actinomycètes par étalement, observation en microscopie optique et reconnaissance visuelle de leurs caractéristiques morphologiques. Les bactéries actinomycètes ainsi isolées et purifiées sont transférées sur milieu Bennett pour clonage. Les colonies isolées sont maintenues à 4°C pendant deux mois, prélevées et mises en suspension dans du glycérol stérile à 20 % puis placées et conservées à -20°C.

Pour analyser la séquence de l'ADNᵣ 16S de bactérie à identifier, on cultive lesdites bactéries en milieu liquide puis on extrait leur ADN génomique et on amplifie -notamment par PCR (« *Polymerase Chain Reaction* »)- sélectivement la séquence ADNᵣ 16S par PCR en utilisant :
- une amorce universelle « 27f » de séquence SEQ ID_NO2 suivante :
   « agagtttgat cctggctcag », et ;
- une amorce universelle « 1492r » de séquence SEQ ID_NO3 suivante :
   « ggttaccttg ttacgactt ». On réalise ensuite un séquençage de l'ADNᵣ 16S par toute méthode connue de l'homme du métier et on compare la séquence de l'ADNᵣ 16S obtenu avec la séquence SEQ ID_NO1.

Pour ce faire, on cultive les bactéries à identifier sous agitation dans 100 mL de milieu ISP-2 (« ISP Médium 2, International *Streptomyces* Project Yeast Malt Extract Agar ») liquide à 30°C pendant 5 jours. On sépare le mycélium obtenu et le milieu de culture par centrifugation et on lave le mycélium deux fois à l'eau bi-distillée. On réalise une lyse du mycélium dans 500 µl de tampon de lyse (Tris-HCl 400 mM, EDTA 60 mM, NaCl 150 mM, SDS 1 %, pH 8,0) pendant 10 min à température ambiante. On ajoute ensuite au milieu de lyse 150 µL d'une solution (pH 4,8) obtenue par mélange de 60 mL d'acétate de potassium 5M, 11,5 mL d'acide acétique glacial et 28,5 d'eau distillée et on agite vigoureusement. On centrifuge à 10000 g pendant 1 min le milieu de lyse obtenu. On collecte le surnageant que l'on soumet à une nouvelle étape de centrifugation à 10000 g pendant 1 min. On collecte le surnageant et on lui adjoint un volume égal d'isopropanol. Après agitation, on centrifuge à 10 000g pendant 2 min. L'ADN précipité est lavé avec 300 µL d'alcool éthylique à 70 %, centrifugé puis séché à l'air et dissout dans 50 µL d'eau bi-distillée stérile.

On réalise la PCR avec un nécessaire (InVitrogen) et selon un profil thermique (« Techne Touch Gene PCR Thermal Cycler ») :
- dénaturation à 98°C pendant 3 min ;
- addition de la « Taq-polymérase » ;
- 30 cycles d'amplification comprenant :
   ▪ une phase de chauffage à 94°C pendant 1 min, suivie de ;
   ▪ une phase de chauffage à 52°C pendant 1 min, suivie de ;
   ▪ une phase de chauffage à 72°C pendant 2 min, suivie de ;
- étape d'extension à 72°C pendant 10 min.

Le produit PCR est analysé et détecté sur gel d'électrophorèse et révélé par le bromure d'éthidium sous lumière ultraviolette. On compare ensuite la séquence de l'ADNᵣ 16S des bactéries à identifier avec la séquence SEQ ID_NO1.

Dans un premier mode de réalisation d'une première variante d'un procédé de traitement d'une matière végétale selon l'invention, on prépare une composition de traitement comprenant au moins une bactérie sous forme végétative et présentant une séquence d'ADN homologue à plus de 99 % -borne exclue- avec la SEQ ID_NO1 par ensemencement d'un milieu de culture, par exemple un milieu de culture liquide ou un milieu de culture solide, avec un inoculum de bactéries de la souche déposée à la CNCM nous le n° I-4467 ou d'au moins l'un de ses mutants présentant la séquence d'ADN SEQ ID_NO1. Le milieu de culture peut être un milieu complet (ou empirique), c'est-à-dire un milieu riche indéfini et comprenant tous les éléments nécessaires à la croissance des bactéries de la souche déposée à la CNCM. On ensemence un volume d'un inoculum de la bactérie déposée à la CNCM dans vingt volumes de milieu complet. On maintient la culture à la température de 30°C sous agitation pendant 5 jours.

Un tel milieu complet pour la production d'une composition de traitement comprenant des bactéries sous forme végétative comprend, par exemple, du D-glucose, un extrait de levure, du phosphate de potassium dibasique (K₂HPO₄), du sulfate d'ammonium ((NH₄)₂SO₄), du chlorure de potassium (KCℓ) et du glycérol à pH 7,2.

Dans un deuxième mode de réalisation d'une première variante d'un procédé de traitement d'une matière végétale selon l'invention, on prépare une composition de traitement comprenant au moins une bactérie sous forme de spores et présentant une séquence d'ADN homologue à 100 % avec la SEQ ID_NO1 par ensemencement d'un milieu de sporulation avec un inoculum de la bactérie déposée à la CNCM nous le n° I-4467 ou de l'un de ses mutants. Le milieu de sporulation peut être, par exemple, un milieu comprenant du D-glucose, un extrait de levure, une peptone, du carbonate de calcium (CaCO₃) et de l'eau distillée à pH 7,2. On maintient la culture à la température de 30°C pendant 6 jours.

Dans une deuxième variante d'un procédé de traitement d'une matière végétale selon l'invention, on prépare une composition acellulaire par centrifugation d'un milieu de culture selon le premier mode de réalisation ou d'un milieu de sporulation selon le deuxième mode de réalisation dans lesquels les bactéries et/ou les spores sont sensiblement éliminées.

### EXEMPLE 1 - Fertilisation

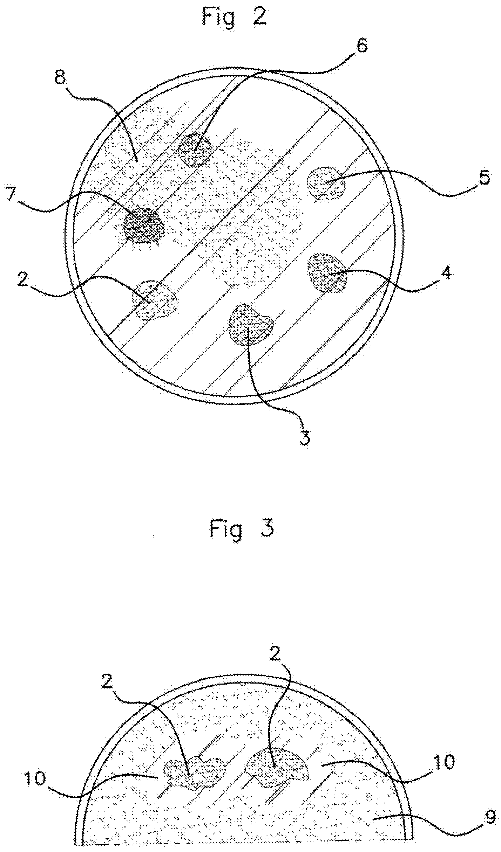

Les bactéries selon l'invention conformes à la souche telle que déposée à la CNCM promeuvent la solubilisation d'éléments nutritionnels solides -notamment du phosphore- d'un milieu de culture. Les inventeurs ont observé (figure 3), dans un milieu de culture solide gélosé 9 opacifié par une poudre de phosphate de calcium, la formation d'un halo translucide 10 entourant les colonies 2 de bactéries selon l'invention attestant de la solubilisation du phosphate de calcium par les bactéries. Les bactéries selon l'invention permettent d'augmenter la dissolution -dans la rhizosphère de végétaux- d'un produit de fertilisation solide inassimilable par lesdits végétaux et d'améliorer la nutrition de végétaux.

### EXEMPLE 2 - Stimulation de la croissance du tournesol et du maïs.

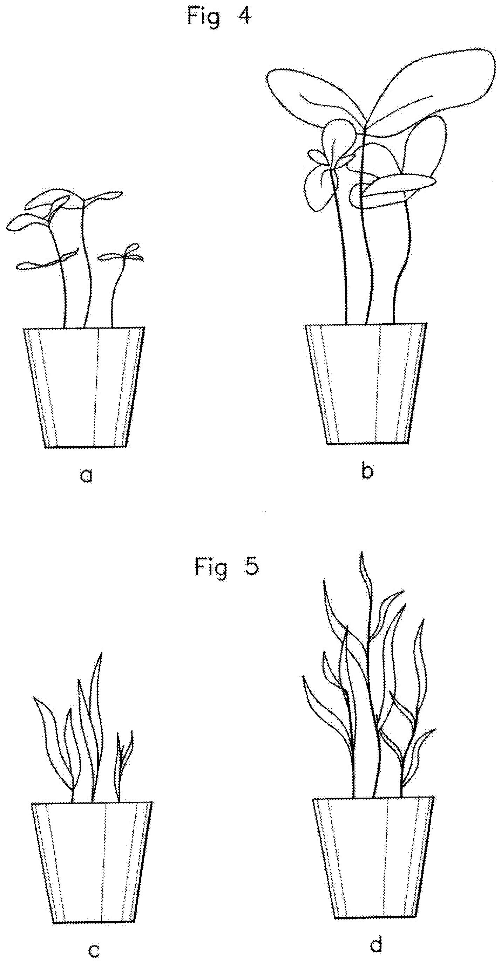

On prépare une composition liquide selon l'invention comprenant des cellules végétatives et des spores de la bactérie selon l'invention que l'on applique par pelliculage sur des graines de tournesol et sur des graines de maïs. La composition liquide comprend entre 1 et 2 g de bactéries par litre de composition, la masse de bactéries étant la masse des bactéries humides. On réalise en parallèle un semis de graines et une culture de plants de tournesol (fig 4a) et de maïs (fig 5c) à titre de contrôles non traités par une composition selon l'invention. Les inventeurs ont aussi observé une stimulation de la croissance initiale des plants de tournesol (figure 4b) et de plants de maïs (figure 5d)-. Les bactéries selon l'invention en application sur les graines permettent de stimuler la croissance de végétaux -en particulier la croissance des parties aériennes de végétaux- tels que le tournesol et le maïs.

### EXEMPLE 3 - Protection de feuilles de vigne vis-à-vis de Botrytis cinerae

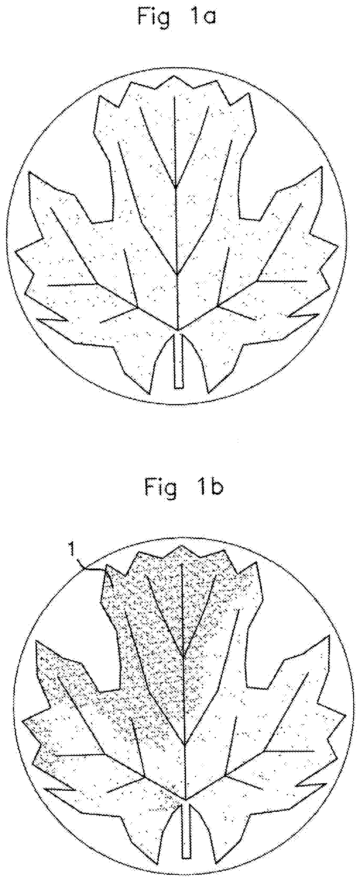

L'application sur des feuilles de vigne de spores de la souche selon l'invention déposée à la CNCM permet de prévenir/éliminer sur les feuilles de vigne prétraitées l'apparition des symptômes (taches brunes sur les feuilles et représentées par un motif grisé 1 sur la figure 1b) dus au champignon *Botrytis cinerae.* La reproduction d'une photographie de feuilles de vigne prétraitées (Fig la) ou non (Fig 1b) avec une composition de spores de la bactérie selon l'invention et infectées par *Botrytis cinerae* est présentée en figure 1. Une telle application permet aussi d'inhiber la germination des spores du champignon *Botrytis cinerae* appliqué ultérieurement sur ces feuilles de vigne et son développement.

### EXEMPLE 4 - Inhibition de la croissance d'un micro-organisme cible.

On détermine et on quantifie l'activité inhibitrice de la croissance d'un micro-organisme cible par la méthode des cylindres (Bauer et al, 1996) dans laquelle on ensemence les bactéries de la souche déposée à la CNCM sur un milieu Bennett gélosé solide et on place ce milieu ensemencé pendant 5 jours à 30°C de façon à former une composition de traitement solide. On prélève un fragment cylindrique, par exemple un fragment cylindrique de 6 mm de diamètre, de la composition de traitement solide et on dépose ce fragment cylindrique en surface d'un milieu de culture ensemencé avec un micro-organisme cible, par exemple un micro-organisme cible phyto-pathogène. Le milieu de culture du micro-organisme cible peut être par exemple un milieu PDA (« *Potato Dextrose Agar* ») pour les champignons phyto-pathogènes ou un milieu Bennett pour les bactéries phyto-pathogènes. On maintient le fragment cylindrique en surface du milieu de culture ensemencé avec le micro-organisme cible pendant 4 heures à la température de 4°C de façon à permettre la diffusion de composés du milieu de culture de la bactérie selon l'invention dans le milieu ensemencé avec le micro-organisme cible.

On place le milieu ensemencé avec le micro-organisme cible pendant 48 heures à 30°C. On mesure le diamètre de la zone d'inhibition de croissance du micro-organisme cible.

La composition de traitement solide selon l'invention présente une activité inhibitrice de la croissance de bactéries et/ou de champignons phyto-pathogènes. Le spectre d'activité de la composition de traitement solide vis-à-vis de la croissance de micro-organismes est présentée au tableau 3 ci-après dans lequel le signe (-) correspond à l'absence d'activité inhibitrice, le signe (+) correspond à un diamètre d'inhibition compris entre 10 mm 15 mm, le signe (++) correspond à un diamètre d'inhibition compris entre 15 mm et 20 mm et le signe (+++) correspond à un diamètre d'inhibition supérieur à 20 mm.

**Tableau 3**

| Micro-organisme cible | Activité inhibitrice |
|---|---|
| *Phaeomoniella chlamydospora* | +++ |
| *Phaeomoniella aelophilum* | +++ |
| *Fomitiporia mediterranea* | ++ |
| *Eutypa lata* | +++ |
| *Botryosphaeria obtusa* | ++ |
| *Botryosphaeria dothidea* | ++ |
| *Botrytis cinerea* | ++ |
| *Verticillium dahliae* | +++ |
| *Fusarium culmorum* | +++ |
| *Pythium ultimum* | ++ |
| *Micrococcus luteus* | +++ |
| *Bacillus subtilis* | +++ |
| *Pseudomonas fluorescens* | - |

Dans ces conditions, le diamètre d'inhibition de la croissance du mycélium de la souche *Botrytis cinerea* par la souche selon l'invention est de 28 mm, le diamètre d'inhibition de la croissance du mycélium de la souche *Fusarium culmorum* est de 30 mm et le diamètre d'inhibition de la croissance du mycélium de la souche *Pythium ultimum* est de 26 mm. Les bactéries selon l'invention présentent avantageusement des capacités inhibitrices de la croissance d'agents phyto-pathogènes de la vigne comme, par exemple, *Phaeomoniella chlamydospora*, *Phaeomoniella aelophilum*, *Eutypa lata*, *Fomitiporia mediterranea* et *Botryosphaeria obtusa.*

La composition de traitement solide comprenant des bactéries de la souche déposée à la CNCM permet de limiter le développement d'agents phyto-pathogènes bactériens ou fongiques.

On visualise en figure 2 l'inhibition à grande distance de la croissance du mycélium 8 du champignon *Phaeomoniella chlamydospora* choisi à titre d'agent pathogène du bois de la vigne par les bactéries 2 de la souche selon l'invention déposée à la CNCM en comparaison avec l'inhibition de la croissance du mycélium 8 du champignon *Phaeomoniella chlamydospora* par des souches 3, 4 et 5 de collection distinctes de la souche selon l'invention. Il est à noter que les souches 7 et 8 de collection n'inhibent pas la croissance du mycélium 8 de l'agent pathogène. Les bactéries selon l'invention permettent de limiter le développement d'agents phyto-pathogènes bactériens ou fongiques.

### EXEMPLE 5 - Stimulation des défenses naturelles (SDN) d'Arabidopsis thaliana.

On met en culture des bactéries du genre *Streptomyces* de la souche déposée à la CNCM sous le n° I-4467 dans un milieu de culture liquide carencé adapté pour favoriser la production de spores et pendant une durée suffisante pour permettre la croissance de ladite souche. On obtient une pré-culture en phase stationnaire de croissance comprenant entre 1 et 2 g de bactéries par litre de pré-culture, la masse de bactéries étant la masse des bactéries humides. On forme ensuite une composition de traitement selon l'invention en diluant un volume de la pré-culture dans 100 volumes d'eau. On met en contact les racines de plantules *d'Arabidopsis thaliana* avec la composition de traitement comprenant des bactéries selon l'invention puis on ensemence lesdites plantules sur un milieu de culture Gamborg B5 gélosé préalablement ensemencé avec une suspension de spores de *Botrtytis cinerae* à titre d'agent phyto-pathogène (à une concentration de 10⁵ unités formatrices de colonies (« colony forming unit, cfu »). On place le milieu de culture ensemencé dans une chambre à 25°C.

Après une semaine, on constate que le traitement préventif selon l'invention des plantules *d'Arabidopsis thaliana* par la composition de traitement comprenant des bactéries selon l'invention permet de prévenir l'effet de *Botrtytis cinerae* en prévenant sa germination (effet systémique) et en stimulant les défenses naturelles des plantules *d'Arabidopsis thaliana* vis-à-vis de *Botrtytis cinerae.* À titre de contrôle, on met en culture des plantules d'*Arabidopsis thaliana* non traitées par la composition de traitement selon l'invention sur un milieu nutritif de culture Gamborg B5 gélosé préalablement ensemencé avec une suspension de spores de *Botrtytis cinerae.*

On mesure l'expression des gènes de défenses des plantules *d'Arabidopsis thaliana* par RT-PCR et en particulier l'expression du gène PR-1 (« Pathogenesis-Related protein of type 1 ») responsable de la synthèse de composés antifongiques chez les plantes. On analyse l'activation des gènes de plantules d'*Arabidopsis thaliana* après dix jours de culture des plantules *d'Arabidopsis thaliana.* Pour ce faire, on extrait les ARN messagers (ARNm) des plantules (du contrôle et du traitement préventif selon l'invention), on convertit ces ARNm en ADNc (ADN complémentaire) en utilisant le kit « SuperScript™ » II RNAse H Reverse Transcriptase (InVitrogen, Carlsbad, USA) et une amorce oligo(dT)22.

On amplifie les ADNc spécifiques de PR-1 par PCR au moyen des amorces spécifiques suivantes :
- « PR-1 f » de séquence SEQ ID_NO4 suivante :
   5'-CTGGCTATTCTCGATTTTTAATCG-3', et ;
- « PR-1 r » de séquence SEQ ID_NO5 suivante :
   5'-TCCTGCATATGATGCTCCTTATTG-3'.

On amplifie en outre, à titre de contrôle d'expression, les ADNc spécifiques du gène EF-1αA4 (Liboz et al, (1990), Plant Mol. Biol., 14, 107-110*. The four members of the gene family encoding the Arabidopsis thaliana translation elongation factor EF-1α are actively transcribed)* par PCR en utilisant les amorces spécifiques suivantes :
- « EF-lf » de séquence SEQ ID_NO6 suivante :
   5'-ATGCCCCAGGACATCGTGATTTCA-3', et ;
- « EF-lr » de séquence SEQ ID_NO7 suivante :
   5'-TTGGCGGCACCCTTAGCTGGATCA-3'.

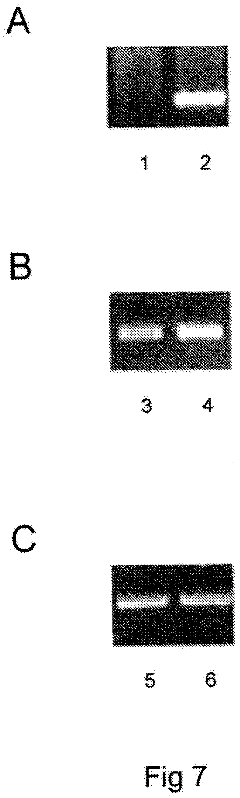

Les produits PCR sont analysés par électrophorèse sur gel et visualisés sous lumière ultraviolette en présence de bromure d'éthidium. Les résultats sont donnés en figure 7. On observe une stimulation de l'expression du gène PR-1 dans les plantules *d'Arabidopsis thaliana* traitées par la composition de traitement (A2) en comparaison avec les plantules contrôle *d'Arabidopsis thaliana* non traitées (A1) par la composition de traitement dans lesquelles l'expression de PR-1 est indétectable.

On observe une stimulation de l'expression (B) du gène PAL 1 dans les plantules *d'Arabidopsis thaliana* traitées par la composition de traitement (B4) en comparaison avec les plantules d'*Arabidopsis thaliana* non traitées (B3) par la composition de traitement, ledit gène PAL 1 étant connu pour constituer un marqueur moléculaire et un contrôle positif de stimulation des défenses naturelles des plantes.

On observe, pour le gène EF1αA4 (contrôle négatif de de normalisation du niveau d'expression génique), une expression sensiblement inchangée dans les plantules *d'Arabidopsis thaliana* (C5) non traitées par la bactérie du genre *Streptomyces et* dans les plantules d'*Arabidopsis thaliana* (C6) traitées par la bactérie du genre *Streptomyces.*

Le traitement des plantules *d'Arabidopsis thaliana* par la bactérie du genre *Streptomyces* conduit à l'activation du gène PR-1, à la production d'antibiotiques végétaux, à la production de phyto-alexines et de composés destinés à renforcer les parois des cellules végétales.

### EXEMPLE 6 - Stimulation des défenses naturelles (SDN) d'Arabidopsis thaliana - stimulation du flux calcique

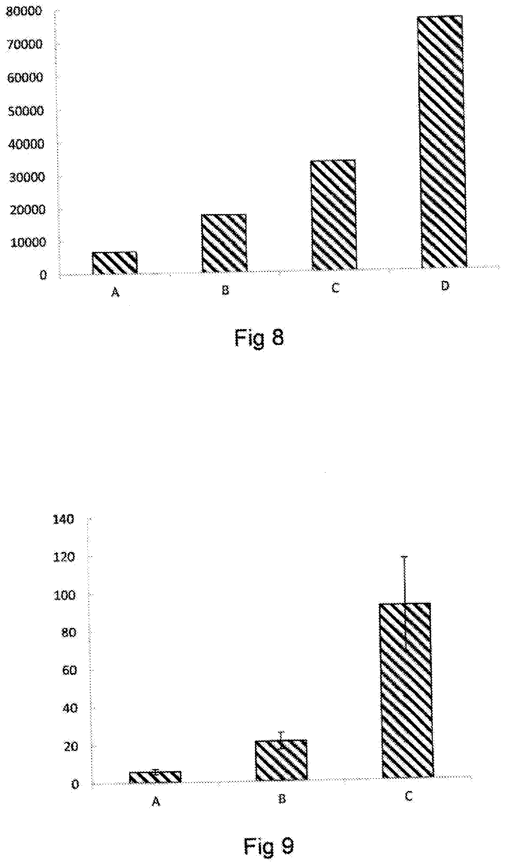

On mesure par luminescence le flux calcique induit par le traitement de plantules *d'Arabidopsis thaliana* avec une composition de traitement obtenue par dilution par un facteur 10x ou par un facteur 100x de la pré-culture décrite à l'exemple 5. Les résultats sont représentés en figure 8. On observe, 20 minutes après le traitement avec la composition de traitement (B) diluée 100x, une augmentation du flux calcique d'un facteur 2,7 par rapport à un traitement à l'eau (A).

On observe aussi, 20 minutes après le traitement avec la composition de traitement (D) diluée 10x, une augmentation du flux calcique d'un facteur 11,6 par rapport à un traitement à l'eau (A) et d'un facteur 2,3 par rapport à un traitement par un contrôle (C) positif d'induction des défenses naturelles des plantes et comprenant un extrait pariétal de l'oomycète pathogène *Phytospora parasitica.*

Les compositions de traitement diluées 10x ou 100x selon l'invention activent les étapes précoces des défenses naturelles de plantes.

### EXEMPLE 7 - Expression de PR-1 à 48 heures après traitement avec une composition de traitement selon l'invention

On analyse par fluorimétrie, à 48 heures après traitement par une composition de traitement diluée 100x telle que décrite à l'exemple 5, le niveau d'expression du gène PR-1 *d'Arabidopsis thaliana* transgénique modèle. Les résultats sont donnés en figure 9. On observe une augmentation du niveau d'expression du gène PR 1 (exprimé en unités de fluorescence par mg de protéine) de la plante traitée par la composition de traitement selon l'invention et diluée 100x (C) d'un facteur 16 par rapport au niveau d'expression du gène PR 1 de la plante traitée par l'eau (A) et d'un facteur 4,4 par rapport au niveau d'expression du gène PR 1 de la plante traitée par un contrôle (B) positif d'induction des défenses naturelles des plantes et d'induction des défenses naturelles des plantes et comprenant un extrait pariétal de l'oomycète pathogène *Phytospora parasitica.*

### EXEMPLE 8 - Protection de plantules d'Arabidopsis thaliana vis-à-vis de Colletotrichum higginsianum

On évalue par luminescence la protection conférée par une composition acellulaire selon l'invention vis-à-vis d'une infection par *Colletotrichum higginsianum* sur des plantules *d'Arabidopsis thaliana* On prépare une composition acellulaire selon l'invention par dilution par un facteur 10x d'une pré-culture de bactéries selon l'invention et comprenant 1mg de biomasse par mL de préculture. On effectue un traitement de thermisation (15' à 90°C) de la composition acellulaire selon l'invention. On applique cette composition acellulaire thermisée pendant 48 heures sur des plantules *d'Arabidopsis thaliana* âgées de 3 semaines puis on inocule les plantules avec *Colletotrichum higginsianum.* On observe une protection vis-à-vis de *Colletotrichum higginsianum* qui est améliorée (1600 rfu) par rapport à un contrôle négatif (2000 rfu). La protection conférée par la composition acellulaire thermisée est sensiblement équivalente à la protection conférée par la composition acellulaire non thermisée.

Il va de soi que l'invention peut faire l'objet de nombreuses variantes de réalisation et applications.

### LISTAGE DE SEQUENCES

SEQ ID_NO1
SEQ ID_NO2
   agagtttgat cctggctcag 20
SEQ ID_NO3
   ggttaccttg ttacgactt 19
SEQ ID_NO4
   ctggctattc tcgattttta atcg 24
SEQ ID_NO5
   tcctgcatat gatgctcctt attg 24
SEQ ID_NO6
   atgccccagg acatcgtgat ttca 24
SEQ ID_NO7
   ttggcggcac ccttagctgg atca 24
SEQ ID_NO8
SEQ ID_NO9
SEQ ID_NO10
SEQ ID_NO11
SEQ ID_NO12

## Revendications

1. Procédé de traitement d'une matière végétale, dans lequel on applique une composition de traitement comprenant au moins un agent biologique choisi dans le groupe formé :
- des bactéries du genre *Streptomyces* comprenant une séquence d'ADN, dit ADNᵣ 16S, codant pour l'ARN ribosomal 16S de ladite bactérie, homologue à 100 % avec la SEQ ID_NO1,
- des milieux de culture comprenant des polynucléotides présentant une séquence d'ADN homologue à 100 % avec la SEQ ID_NO1, obtenus par :
. mise en culture d'au moins une bactérie du genre *Streptomyces* comprenant la séquence d'ADNr 16S homologue à 100 % avec la SEQ ID_NO1 dans un milieu de culture apte à permettre la croissance desdites bactéries pendant une durée supérieure à 24 heures, puis ;
. élimination desdites bactéries du milieu de culture de façon à former une composition de traitement au moins sensiblement exempte desdites bactéries ;
les bactéries du genre *Streptomyces* étant choisies dans le groupe formé :
. des bactéries conformes à la souche déposée et enregistrée en date du 7 avril 2011 sous le numéro I-4467 auprès de la Collection Nationale de Cultures de Microorganismes (CNCM) de l'Institut Pasteur, et ;
. des bactéries mutantes obtenues par mutagenèse de bactéries conformes à la souche déposée et enregistrée en date du 7 avril 2011 sous le numéro I-4467 auprès de la Collection Nationale de Cultures de Microorganismes (CNCM) de l'Institut Pasteur, les bactéries mutantes étant inhibitrices de la croissance d'agents phytopathogènes de la vigne comme *Phaeomoniella chlamydospora*, *Phaeomoniella aelophilum, Eutypa lata*, *Fomitiporia mediterranea* et *Botryosphaeria obtusa*.

2. Procédé selon la revendication 1, **caractérisé en ce que** la matière végétale est choisie dans le groupe formé de tout ou partie d'un végétal en culture, d'un fruit ou d'un légume après récolte, de semences de végétaux.

3. Procédé selon l'une des revendications 1 ou 2, **caractérisé en ce qu'**on utilise une composition de traitement comprenant en outre un nutriment solide à l'état divisé.

4. Procédé selon l'une des revendications 1 à 3, **caractérisé en ce qu'**on applique ladite composition de traitement sur ladite matière végétale pour activer la croissance de cette dernière.

5. Procédé selon l'une des revendications 1 à 4, **caractérisé en ce qu'**on applique ladite composition de traitement sur ladite matière végétale pour inhiber la croissance d'au moins un microorganisme cible.

6. Procédé selon l'une des revendications 1 à 5, **caractérisé en ce qu'**on applique ladite composition de traitement sur ladite matière végétale pour stimuler les défenses naturelles de ladite matière végétale.

7. Composition comprenant au moins un agent biologique choisi dans le groupe formé :
- des bactéries du genre *Streptomyces* comprenant une séquence d'ADN, dit ADNr 16S, codant pour l'ARN ribosomal 16S de ladite bactérie homologue à 100 % avec la SEQ ID_NO1, et ;
- des milieux de culture dans lesquels se sont développées des bactéries du genre *Streptomyces* comprenant la séquence d'ADNr 16S homologue à 100 % avec la SEQ ID_NO1 et qui sont sensiblement exempts desdites bactéries, lesdits milieux de culture comprenant des polynucléotides présentant une séquence d'ADN homologue à 100 % avec la SEQ ID_NO1 ;
les bactéries du genre *Streptomyces* étant choisies dans le groupe formé :
. des bactéries conformes à la souche déposée et enregistrée en date du 7 avril 2011 sous le numéro I-4467 auprès de la Collection Nationale de Cultures de Microorganismes (CNCM) de l'Institut Pasteur, et ;
. de bactéries mutantes obtenues par mutagenèse de bactéries conformes à la souche déposée et enregistrée en date du 7 avril 2011 sous le numéro I-4467 auprès de la Collection Nationale de Cultures de Microorganismes (CNCM) de l'Institut Pasteur, les bactéries mutantes étant inhibitrices de la croissance d'agents phytopathogènes de la vigne comme *Phaeomoniella chlamydospora*, *Phaeomoniella aelophilum, Eutypa lata*, *Fomitiporia mediterranea* et *Botryosphaeria obtusa*.

8. Composition selon la revendication 7, **caractérisée en ce qu'**elle est liquide.

9. Composition selon la revendication 7, **caractérisée en ce qu'**elle est solide.

10. Composition selon l'une des revendications 7 à 9, **caractérisée en ce qu'**elle comprend au moins un excipient acceptable pour permettre son application sur une matière végétale à traiter.

11. Utilisation en agriculture d'une composition selon l'une des revendications 7 à 10.

12. Utilisation selon la revendication 11 pour stimuler la croissance de végétaux.

13. Utilisation selon la revendication 11 pour stimuler la germination de graines.

14. Utilisation selon la revendication 11 pour un traitement de fertilisation de végétaux.

15. Utilisation selon la revendication 11 pour un traitement antimicrobien et/ou antiviral de végétaux.

16. Utilisation selon la revendication 11 pour stimuler les défenses naturelles des végétaux.

## Patentansprüche

1. Verfahren zur Behandlung eines pflanzlichen Materials, wobei eine Behandlungszusammensetzung angewendet wird, umfassend mindestens einen biologischen Stoff, ausgewählt aus der Gruppe, gebildet aus:
- Bakterien der Gattung *Streptomyces,* umfassend eine ADN-Sequenz, bezeichnet als ADNᵣ 16S, die für die ribosomale ARN 16S der Bakterie codiert, die zu 100 % mit der SEQ ID_NO1 homolog ist,
- Kulturmedien, umfassend Polynucleotide, die eine ADN-Sequenz aufweisen, die zu 100 % mit der SEQ ID_NO1 homolog ist, erhalten durch:
• Kultivieren von mindestens einer Bakterie der Gattung *Streptomyces,* umfassend die ADNr 16S-Sequenz, die zu 100 % mit der SEQ ID_NO1 homolog ist, in einem Kulturmedium, das ausgelegt ist, um das Wachstum der Bakterien während einer Dauer von mehr als 24 Stunden zu ermöglichen, dann;
• Entfernen der Bakterien aus dem Kulturmedium, um eine Behandlungszusammensetzung zu bilden, die mindestens im Wesentlichen frei von den Bakterien ist;
wobei die Bakterien der Gattung *Streptomyces* ausgewählt sind aus der Gruppe, gebildet aus:
• Bakterien entsprechend dem Stamm, hinterlegt und registriert am 7. April 2011 unter der Nummer 1-4467 in der Collection Nationale de Culture de Microorganismes (CNCM) beim Institut Pasteur, und;
• mutanten Bakterien, erhalten durch Mutagenese von Bakterien, entsprechend dem Stamm, hinterlegt und registriert am 7. April 2011 unter der Nummer I-4467 in der Collection Nationale de Culture de Microorganismes (CNCM) beim Institut Pasteur, wobei die mutanten Bakterien das Wachstum von phytopatogenen Stoffen des Weins wie z. B. *Phaeomoniella chlamydospora*, *Phaeomoniella aelophilum, Eutypa lata*, *Fomitiporia mediterranea* und *Botryosphaeria obtusa* verhindern.

2. Verfahren nach Anspruch 1, **dadurch gekennzeichnet, dass** das pflanzliche Material ausgewählt ist aus der Gruppe, gebildet aus der Gesamtheit oder einem Teil eine Kulturpflanze, einer Frucht oder einem Gemüse nach der Ernte, von Pflanzensaatgut.

3. Verfahren nach einem der Ansprüche 1 oder 2, **dadurch gekennzeichnet, dass** eine Behandlungszusammensetzung verwendet wird, die außerdem einen festen Nährstoff in getrenntem Zustand umfasst.

4. Verfahren nach einem der Ansprüche 1 bis 3, **dadurch gekennzeichnet, dass** die Behandlungszusammensetzung auf das pflanzliche Material angewendet wird, um das Wachstum dieses Letzteren zu aktivieren.

5. Verfahren nach einem der Ansprüche 1 bis 4, **dadurch gekennzeichnet, dass** die Behandlungszusammensetzung auf das pflanzliche Material angewendet wird, um das Wachstum von mindestens einem Ziel-Mikroorganismus zu hemmen.

6. Verfahren nach einem der Ansprüche 1 bis 5, **dadurch gekennzeichnet, dass** die Behandlungszusammensetzung auf das pflanzliche Material angewendet wird, um die natürlichen Abwehrkräfte des pflanzlichen Materials zu stimulieren.

7. Zusammensetzung, umfassend mindestens einen biologischen Stoff, ausgewählt aus der Gruppe, gebildet aus:
- Bakterien der Gattung *Streptomyces,* umfassend eine ADN-Sequenz, bezeichnet als ADNᵣ 16S, die für die ribosomale ARN 16S der Bakterie codiert, die zu 100 % mit der SEQ ID_NO1 homolog ist, und;
- Kulturmedien, in denen Bakterien der Gattung *Streptomyces* entwickelt werden, umfassend die ADNᵣ 16S-Sequenz, die zu 100 % mit der SEQ ID_NO1 homolog ist, und die im Wesentlichen frei von den Bakterien sind, wobei die Kulturmedien Polynucleotide umfassen, die eine ADN-Sequenz aufweisen, die zu 100 % mit der SEQ ID_NO1 homolog ist;
wobei die Bakterien der Gattung *Streptomyces* ausgewählt sind aus der Gruppe, gebildet aus:
• Bakterien entsprechend dem Stamm, hinterlegt und registriert am 7. April 2011 unter der Nummer I-4467 in der Collection Nationale de Culture de Microorganismes (CNCM) beim Institut Pasteur, und;
• mutanten Bakterien, erhalten durch Mutagenese von Bakterien, entsprechend dem Stamm, hinterlegt und registriert am 7. April 2011 unter der Nummer I-4467 in der Collection Nationale de Culture de Microorganismes (CNCM) beim Institut Pasteur, wobei die mutanten Bakterien das Wachstum von phytopatogenen Stoffen des Weins wie z. B. *Phaeomoniella chlamydospora*, *Phaeomoniella aelophilum, Eutypa lata*, *Fomitiporia mediterranea* und *Botryosphaeria obtusa* verhindern.

8. Zusammensetzung nach Anspruch 7, **dadurch gekennzeichnet, dass** sie flüssig ist.

9. Zusammensetzung nach Anspruch 7, **dadurch gekennzeichnet, dass** sie fest ist.

10. Zusammensetzung nach einem der Ansprüche 7 bis 9, **dadurch gekennzeichnet, dass** sie mindestens einen annehmbaren Trägerstoff umfasst, um ihre Anwendung auf ein zu behandelndes pflanzliches Material zu ermöglichen.

11. Verwendung einer Zusammensetzung nach einem der Ansprüche 7 bis 10 in der Landwirtschaft.

12. Verwendung nach Anspruch 11 zum Stimulieren des Wachstums von Pflanzen.

13. Verwendung nach Anspruch 11 zum Stimulieren der Keimung von Samen.

14. Verwendung nach Anspruch 11 zur Befruchtungsbehandlung von Pflanzen.

15. Verwendung nach Anspruch 11 zur antimikrobiellen und/oder antiviralen Behandlung von Pflanzen.

16. Verwendung nach Anspruch 11 zum Stimulieren der natürlichen Abwehrkräfte der Pflanzen.

## Claims

1. Method for treating a plant material, wherein a treatment composition is applied comprising at least one biological agent chosen in the group composed of:
- *Streptomyces* genus bacteria comprising a DNA sequence, referred to as 16S rDNA, coding for the 16S ribosomal RNA of said bacteria, 100% homologous with SEQ ID_NO1,
- culture media comprising polynucleotides having a DNA sequence 100% homologous with SEQ ID_NO1, obtained by:
• culturing at least one *Streptomyces* genus bacterium comprising the 16S rDNA sequence 100% homologous with SEQ ID_NO1 in a culture medium suitable for enabling the growth of said bacteria for a duration greater than 24 hours, then;
• removing said bacteria from the culture medium so as to form a treatment composition at least substantially free from said bacteria;
the *Streptomyces* genus bacteria being chosen in the group composed of:
• bacteria complying with the strain deposited and registered on 7 April 2011 under the number 1-4467 with the Institut Pasteur National Collection of Micro-organism Cultures (CNCM), and;
• mutant bacteria obtained by mutagenesis of bacteria complying with the strain deposited and registered on 7 April 2011 under the number 1-4467 with the Institut Pasteur National Collection of Micro-organism Cultures (CNCM), the mutant bacteria inhibiting the growth of grapevine phytopathogenic agents such as *Phaeomoniella chlamydospora*, *Phaeomoniella aelophilum, Eutypa* lata, *Fomitiporia mediterranea* and *Botryosphaeria obtusa*.

2. Method according to claim 1, **characterised in that** the plant material is chosen in the group composed of all or part of a crop plant, fruit or vegetable after harvesting, plant seeds.

3. Method according to one of claims 1 or 2, **characterised in that** a treatment composition further comprising a solid nutrient in the divided state is used.

4. Method according to one of claims 1 to 3, **characterised in that** said treatment composition is applied on said plant material to activate the growth thereof.

5. Method according to one of claims 1 to 4, **characterised in that** said treatment composition is applied on said plant material to inhibit the growth of at least one target micro-organism.

6. Method according to one of claims 1 to 5, **characterised in that** said treatment composition is applied on said plant material to stimulate the natural defences of said plant material.

7. Composition comprising at least one biological agent chosen from the group composed of:
- *Streptomyces* genus bacteria comprising a DNA sequence, referred to as 16S rDNA, coding for the 16S ribosomal RNA of said bacteria, 100% homologous with SEQ ID_NO1, and;
- culture media wherein *Streptomyces* genus bacteria comprising the 16S rDNA sequence 100% homologous with SEQ ID_NO1 have been grown and which are substantially free from said bacteria, said culture media comprising polynucleotides having a DNA sequence 100% homologous with SEQ ID_NO1;
the *Streptomyces* genus bacteria being chosen in the group composed of:
• bacteria complying with the strain deposited and registered on 7 April 2011 under the number 1-4467 with the Institut Pasteur National Collection of Micro-organism Cultures (CNCM), and;
• mutant bacteria obtained by mutagenesis of bacteria complying with the strain deposited and registered on 7 April 2011 under the number 1-4467 with the Institut Pasteur National Collection of Micro-organism Cultures (CNCM), the mutant bacteria inhibiting the growth of grapevine phytopathogenic agents such as *Phaeomoniella chlamydospora*, *Phaeomoniella aelophilum, Eutypa lata, Fomitiporia mediterranea* and *Botryosphaeria obtusa*.

8. Composition according to claim 7, **characterised in that** it is liquid.

9. Composition according to claim 7, **characterised in that** it is solid.

10. Composition according to one of claims 7 to 9, **characterised in that** it comprises at least one acceptable excipient for enabling the application thereof on a plant material to be treated.

11. Use in agriculture of a composition according to one of claims 7 to 10.

12. Use according to claim 11 to stimulate plant growth.

13. Use according to claim 11 to stimulate seed germination.

14. Use according to claim 11 for a plant fertilisation treatment.

15. Use according to claim 11 for an antimicrobial and/or antiviral plant treatment.

16. Use according to claim 11 to stimulate the natural defences of plants.
